# EUROPEAN PATENT SPECIFICATION

(11) **EP 3 377 107 B1**
(45) Date of publication and mention of the grant of the patent: **12.08.2020**
(21) Application number: 16806416.0
(22) Date of filing: 18.11.2016
(51) Int. Cl.: A61K 45/06, A61K 39/395, A61K 31/437, A61K 31/4523, A61K 39/00, C07K 16/28, A61P 35/00, A61P 35/04

(54) **METHODS OF TREATING CANCER USING B-RAF INHIBITORS AND IMMUNE CHECKPOINT INHIBITORS**
VERFAHREN ZUR KREBSBEHANDLUNG MIT B-RAF-HEMMERN UND IMMUNCHECKPOINT-HEMMERN
MÉTHODES DE TRAITEMENT DU CANCER AU MOYEN D'INHIBITEURS DE B-RAF ET D'INHIBITEURS DE POINT DE CONTRÔLE IMMUNITAIRES

(30) Priority: 19.11.2015 US 201562257645 P
(43) Date of publication of application: 26.09.2018
(73) Proprietor: F. Hoffmann-La Roche AG, 4070 Basel (CH)
(72) Inventor: COLBURN, Dawn, 4070 Basel (US); RICHIE, Nicole, 4070 Basel (US)
(74) Representative: Beyermann, Jochen Carl
(86) International application number: PCT/US2016/062859
(87) International publication number: WO 2017/087851

(56) References cited:
- WO-A1-2014/027056
- WO-A1-2014/193898
- WO-A1-2014/195852
- Z. A. COOPER ET AL: "Response to BRAF Inhibition in Melanoma Is Enhanced When Combined with Immune Checkpoint Blockade", CANCER IMMUNOLOGY RESEARCH, vol. 2, no. 7, 29 April 2014 (2014-04-29), pages 643-654, XP055345831, US ISSN: 2326-6066, DOI: 10.1158/2326-6066.CIR-13-0215
- ZACHARY A. COOPER ET AL: "Combining checkpoint inhibitors and BRAF-targeted agents against metastatic melanoma", ONCOIMMUNOLOGY, vol. 2, no. 5, E24320, 1 May 2013 (2013-05-01), page 1, XP055133315, ISSN: 2162-4011, DOI: 10.4161/onci.24320
- Anonymous: "A Phase 1b Study of MPDL3280A (an Engineered Anti-PDL1 Antibody) in Combination With Vemurafenib (Zelboraf®) or Vemurafenib Plus Cobimetinib in Patients With Previously Untreated BRAFV600-Mutation Positive Metastatic Melanoma", ClinicalTrials.gov , 17 February 2015 (2015-02-17), XP002767276, Retrieved from the Internet: URL:https://clinicaltrials.gov/archive/NCT 01656642/2015_02_17 [retrieved on 2017-02-15]
- TERESA KIM ET AL: "Combining targeted therapy and immune checkpoint inhibitors in the treatment of metastatic melanoma", CANCER BIOLOGY & MEDICINE, TIANJIN YIKE DAXUE FUSHU ZHONGLIU YIYUAN,TIANJIN MEDICAL UNIVERSITY CANCER INSTITUTE AND HOSPITAL, CN, vol. 11, no. 4, 1 December 2014 (2014-12-01), pages 237-246, XP002759549, ISSN: 2095-3941, DOI: 10.7497/J.ISSN.2095-3941.2014.04.002
- P. Hwu et al.: "1109PD - Preliminary safety and clinical activity of atezolizumab combined with cobimetinib and vemurafenib in BRAF V600-mutant metastatic melanoma", , 10 October 2016 (2016-10-10), XP002767277, Retrieved from the Internet: URL:http://oncologypro.esmo.org/Meeting-Re sources/ESMO-2016/Preliminary-safety-and-c linical-activity-of-atezolizumab-combined- with-cobimetinib-and-vemurafenib-in-BRAF-V 600-mutant-metastatic-melanoma [retrieved on 2017-02-14]
- SULLIVAN RYAN J ET AL: "Atezolizumab plus cobimetinib and vemurafenib in-mutated melanoma patients", NATURE MEDICINE, NATURE PUB. CO, NEW YORK, vol. 25, no. 6, 1 June 2019 (2019-06-01), pages 929-935, ISSN: 1078-8956, DOI: 10.1038/S41591-019-0474-7 [retrieved on 2019-06-06]

## Description

### FIELD

Provided herein are therapies including dosage regimens for the treatment of cancer using B-RAF and an immune checkpoint inhibitor in combination with a MEK inhibitor.

### BACKGROUND

In 2014, an estimated 76,100 new cases of melanoma will be diagnosed and approximately 9,710 patients will die of the disease in the United States (American Cancer Society 2013). Globally in 2008, the age-standardized rates of incidence and mortality for melanoma were 9.5 and 1.8 per 100,000 among males and 8.6 and 1.1 per 100,000 among females, respectively. The incidence of melanoma is particularly high among Caucasian populations in Australia (42.4 per 100,000) and Western Europe (10.6 per 100,000). Moreover, the incidence of melanoma continues to increase; it is increasing in men more rapidly than any other malignancy and in women more rapidly than any other malignancy with the exception of lung cancer. The lifetime risk of developing melanoma in 2005 was approximately one in 55, with the median age at diagnosis being 59 years. Melanoma ranks second to adult leukemia in terms of loss of years of potential life, per death. Metastatic melanoma accounts for approximately 4% of all newly diagnosed melanoma cases. However, survival for this patient group remains extremely poor, with a 5-year relative survival rate of 15.3%.

Until recently there were a limited number of treatments available for metastatic melanoma. Dacarbazine was considered the standard first-line treatment with response rates of 5%-12%, median progression-free survival (PFS) of less than 2 months, and a median overall survival (OS) of 6.4 to 9.1 months. Combination chemotherapy and chemotherapy combined with interferon (IFN)-alpha (IFN-α) or interleukin-2 (IL-2), while showing improved response rates, have not resulted in improved OS. More recently, newer agents that have the potential of improving outcomes in patients with metastatic melanoma have been investigated. Ipilimumab (Yervoy®), a humanized immunoglobulin (Ig)G1 monoclonal antibody that blocks cytotoxic T-lymphocyte-associated antigen 4 (CTLA-4), was approved by the U.S. Food and Drug Administration (FDA) in 2011 for the treatment of unresectable or metastatic melanoma on the basis of two Phase III studies, which showed improvements in OS compared with glycoprotein 100 (gp100) peptide vaccination and in combination with dacarbazine compared with dacarbazine plus placebo in previously untreated advanced melanoma patients. In these studies, the overall response rate (10.9%) and the median PFS (2.8 months) were within the range historically observed with dacarbazine. However, the 3-year survival rate with ipilimumab plus dacarbazine was 20.8%, indicating that a subset of patients experienced durable clinical.

Vemurafenib (Zelboraf®), a specific inhibitor of V600-mutant forms of BRAF, which are prevalent in approximately 50% of cutaneous melanomas, was approved by the FDA in 2011 for the treatment of BRAFV600 mutation-positive, inoperable, or metastatic melanoma. In patients with previously untreated metastatic melanoma with the BRAFV600E mutation, vemurafenib, compared with dacarbazine, demonstrated a 63% reduction in the risk of death and a 74% improvement in PFS. Response rates were 48% for vemurafenib and 5% for dacarbazine. Updated OS and PFS results continued to demonstrate the clinical benefit of vemurafenib treatment. With a median follow-up of 12.5 months, vemurafenib treatment was associated with a nearly 4-month improvement in median OS (13.6 vs. 9.7 months). The hazard ratio for death in this analysis was 0.70 (95% CI: 0.57, 0.87) in favor of vemurafenib.

Despite prolongation of PFS and OS, disease recurrence occurs in almost all patients treated with BRAF inhibitors. MEK inhibitors have emerged as an additional therapeutic option for the treatment of BRAF-mutated melanoma. MEK is a downstream component of the BRAF/MEK/ERK signaling pathway, which is believed to be upregulated in response to BRAF-inhibition. Additional data have shown that combined BRAF and MEK inhibition can provide a greater clinical benefit compared with BRAF inhibitor monotherapy as well as potentially reduce BRAF inhibitionassociated side effects. Cobimetinib (GDC-0973, R05514041, XL518) is a MEK inhibitor under active clinical investigation and recently approved by the FDA.

Recently, cancer immunotherapy has advanced the treatment landscape in metastatic melanoma. Both nivolumab and pembrolizumab, programmed death-1 (PD-1) receptor- blocking antibodies, received accelerated approval in the United States for the treatment of patients with unresectable or metastatic melanoma and disease progression following ipililmumab and, if BRAFV600 mutation-positive, a BRAF inhibitor contingent upon verification of clinical benefit in confirmatory trials (see the KEYTRUDA® [pembrolizumab] and OPDIVO® [nivolumab] U.S. Package Inserts). Atezolizumab (also known as MPDL3280A) is a humanized IgG1 monoclonal antibody which targets human programmed death-ligand 1 (PD-L1) and inhibits its interaction with its receptor, PD-1. Atezolizumab also blocks the binding of PD-L1 to B7-1, an interaction that is reported to provide additional inhibitory signals to T cells.

Among the goals for new therapies is the challenge to combine the high response rate observed with B-RAF and MEK inhibitors in B-RAF V600-mutant patients with the prolonged duration of response that may be elicited with immune modulation. Inhibition of BRAFV600 by vemurafenib and dabrafenib appears to have relatively little effect on other kinases, and in vitro/ in vivo modeling has suggested that BRAF inhibition improves T lymphocyte recognition of melanoma antigens and increases the numbers of CD4+ and CD8+ tumor infiltrating lymphocytes observed in melanoma tumors. In contrast to BRAFV600E inhibition, MEK inhibition may have a negative impact on T-cell. Luke, Journal American Journal of Hematology / Oncology 11(2):34-38 (February 2015). Further, PD-L1 expression as a mechanism of resistance to BRAF inhibitors because BRAF-resistant cell lines expressed high PD-L1, and the addition of MEK inhibitors has a suppressive effect on PD-L1 expression. See Kim et al., Cancer Biol. Med 11(4):237-246 (2014). Cooper et al. , Cancer Immunology Research 2(7): 643-654 (2014) teaches that administration of a checkpoint inhibitor (anti-CTLA4, anti-PD-1 and anti-PD-L1) together with a B-RAF inhibitor (BRAFi) led to an enhanced response, significantly prolonging survival and slowing tumor growth in patient suffering from melanoma. Continued development of novel treatments is ongoing, as well as pursuance of the optimal treatment regimen (combination on sequence) that will provide the maximum treatment benefit for patients. Thus, clinical trials remain a valid option for the initial treatment of advanced melanoma.

### SUMMARY

Provided herein are combinations comprising a B-RAF inhibitor and a immune checkpoint inhibitor with a MEK inhibitor as defined in the claims. Disclosed herein are methods of treating cancer in an individual, the method comprising first administering to the individual an effective amount of a B-RAF inhibitor and second administering to the individual an effective amount of the B-RAF inhibitor and an effective amount of an immune checkpoint inhibitor.

Also disclosed herein are methods of increasing efficacy of a cancer treatment, the method comprising first administering to the individual an effective amount of a B-RAF inhibitor and second administering to the individual an effective amount of the B-RAF inhibitor and an effective amount of an immune checkpoint inhibitor.

Disclosed herein are also methods of treating cancer in an individual wherein cancer treatment comprises first administering to the individual an effective amount of a B-RAF inhibitor and second administering to the individual an effective amount of the B-RAF inhibitor and an effective amount of immune checkpoint inhibitor, wherein the cancer treatment has increased efficacy compared to administering to the individual an effective amount of the B-RAF inhibitor and an effective amount of the immune checkpoint inhibitor alone.

The B-RAF inhibitor is propane-1-sulfonic acid {3-[5-(4-chlorophenyl)-1H-pyrrolo[2,3-b]pyridine-3-carbonyl]-2,4-difluoro-phenyl}-amide or a pharmaceutically acceptable salt thereof. According to the present invention, the B-RAF inhibitor is vemurafenib. In some embodiments of any of the methods, the first administration and second administration of the B-RAF inhibitor is at a dosage of about 960 mg twice daily, about 720 mg twice daily, and/or about 480 mg twice daily. In some embodiments of any of the methods, the first administration of the B-RAF inhibitor is at a greater dosage then the second administration of the B-RAF inhibitor. In some embodiments of any of the methods, the first administration of the B-RAF inhibitor comprises a first dosage and a second dosage of the B-RAF inhibitor and the first dosage is greater than the second dosage. In some embodiments of any of the methods, the first administration of the B-RAF inhibitor is about 28 days or about 56 days. In some embodiments of any of the methods, the first administration of the B-RAF inhibitor comprises a first dosage and a second dosage of the B-RAF inhibitor, the first dosage is greater than the second dosage, and the first dosage is administered for 21 days and the second dosage is administered for 7 days. In some embodiments of any of the methods, the B-RAF inhibitor is administered orally.

The immune checkpoint inhibitor is a PD-1 axis binding antagonist. The PD-1 axis binding antagonist is an anti-PD-Ll antibody. According to the present invention, the anti-PD-L1 antibody is atezolizumab. In some embodiments, atezolizumab is administered at a dosage of about 15 mg/kg q3w, about 20 mg/kg q3w, about 800 mg q2w, or about 1200 mg q3w. In some embodiments, the immune checkpoint inhibitor is administered intravenously.

According to the present invention, the first administration and the second administration further comprise an effective amount of a MEK inhibitor. The MEK inhibitor is (S)-[3,4-difluoro-2-(2-fluoro-4-iodophenylamino)phenyl] [3-hydroxy-3-(piperidin-2-yl)azetidin-1-yl]methanone or a pharmaceutically acceptable salt thereof. In some embodiments, the MEK inhibitor is (S)-[3,4-difluoro-2-(2-fluoro-4-iodophenylamino)phenyl] [3-hydroxy-3-(piperidin-2-yl)azetidin-1-yl]methanone, hemifumarate. According to the present invention, the MEK inhibitor is cobimetinib. In some embodiments of any of the methods, the first administration and second administration of the MEK inhibitor is at a dosage of about 60 mg daily on a 21 days on/7 days off schedule or about 40 mg daily on a 21 days on/7 days off schedule. In some embodiments of any of the methods, the first administration of the MEK inhibitor is about 28 day schedule. In some embodiments of any of the methods, the MEK inhibitor is administered orally.

In some embodiments of any of the methods, the cancer is melanoma. In some embodiments of any of the methods, the melanoma is unresectable or metastatic melanoma. In some embodiments of any of the methods, the melanoma is B-RAF V600 mutant melanoma. In some embodiments of any of the methods, the B-RAF V600E mutant melanoma or B-RAF V600K mutant melanoma.

Disclosed herein are further methods of treating melanoma in an individual, the method comprising first administering to the individual vemurafenib on a 28 day schedule, wherein vemurafenib is administered at a dosage of 960 mg twice a day for 21 days and then 720 mg twice a day for 7 days of the 28 day schedule and second administering to the individual vemurafenib and atezolizumab, wherein vemurafenib is administered at a dosage of 720 mg twice a day and atezolizumab is administered at a dosage of 1200 mg q3w. In some embodiments, vemurafenib is administered orally as a tablet. In some embodiments, the atezolizumab is administered intravenously. In some embodiments, the cancer is melanoma. In some embodiments, the melanoma is unresectable or metastatic melanoma. In some embodiments, the melanoma is B-RAF V600 mutant melanoma. In some embodiments, the B-RAF V600E mutant melanoma or B-RAF V600K mutant melanoma.

Provided herein are also methods of treating melanoma in an individual, the method comprising first administering to the individual vemurafenib and cobimetinib on a 28 day schedule, wherein vemurafenib is administered at a dosage of 960 mg twice a day for 21 days and then 720 mg twice a day for 7 days of the 28 day schedule and cobimetinib is administered at a dosage of 60 mg daily for 21 days and 7 days off of the 28 day schedule and second administering to the individual vemurafenib, cobimetinib, and atezolizumab, wherein vemurafenib is administered at a dosage of 720 mg twice a day, cobimetinib is administered at a dosage of 60 mg daily for 21 days on and 7 days off, and atezolizumab is administered at a dosage of 800 mg q2w. In some embodiments, vemurafenib is administered orally as a tablet. In some embodiments, the cobimetinib is administered orally as a tablet. In some embodiments, the atezolizumab is administered intravenously. In some embodiments, the cancer is melanoma. In some embodiments, the melanoma is unresectable or metastatic melanoma. In some embodiments, the melanoma is B-RAF V600 mutant melanoma. In some embodiments, the B-RAF V600 mutant melanoma is B-RAF V600E mutant melanoma or B-RAF V600K mutant melanoma.

### DETAILED DESCRIPTION

The invention is defined by the claims. Any subject-matter falling outside the scope of the claims is provided for information purposes only.

### I. Definitions

The term "RAF" and "RAF kinase" refer to a family of three RAF kinases that are serine/threonine-specific protein kinases. The RAF kinases are A-Raf, BRAF and CRAF. The RAF kinases are related to retroviral oncogenes. RAF is an acronym for Rapidly Accelerated Fibrosarcoma.

The term "B-RAF", as used herein, refers, unless indicated otherwise, to any native or variant (whether native or synthetic) B-RAF polypeptide. The term "wild type B-RAF" generally refers to a polypeptide comprising the amino acid sequence of a naturally occurring B-RAF protein. *See e.g.,* Uni. Prot No. P15056 visited on 11.1615. The term "V600 mutant B-RAF" or "V600 mutant B-RAF" generally refers to a b-Raf polypeptide comprising a missense mutation at amino acid V600, *e.g.,* B-RAF V600E or B-RAF V600K.

An "antagonist" (interchangeably termed "inhibitor") of a polypeptide of interest is a molecule that interferes with activation or function of the polypeptide of interest, e.g., partially or fully blocks, inhibits, or neutralizes a biological activity mediated by a polypeptide of interest. For example, an antagonist of polypeptide X may refers to any molecule that partially or fully blocks, inhibits, or neutralizes a biological activity mediated by polypeptide X. Preferably, the inhibitor is a small molecule which binds to the polypeptide of interest. In a particular embodiment, an inhibitor has a binding affinity (dissociation constant) to the polypeptide of interest of about 1,000 nM or less. In another embodiment, inhibitor has a binding affinity to the polypeptide of interest of about 100 nM or less. In another embodiment, an inhibitor has a binding affinity to the polypeptide of interest of about 50 nM or less.. In a particular embodiment, an inhibitor inhibits signaling of the polypeptide of interest with an IC₅₀ of 1,000 nM or less. In another embodiment, an inhibitor inhibits signaling of the polypeptide of interest with an IC₅₀ of 500 nM or less. In another embodiment, an inhibitor inhibits signaling of the polypeptide of interest with an IC₅₀ of 50 nM or less. In certain embodiments, the antagonist reduces or inhibits, by at least about any of 10%, 20%, 30%, 40%, 50%, 60%, 70%, 80%, 90% or more, the expression level or biological activity of the polypeptide of interest.

The term "PD-1 axis binding antagonist" refers to a molecule that inhibits the interaction of a PD-1 axis binding partner with either one or more of its binding partner, so as to remove T-cell dysfunction resulting from signaling on the PD-1 signaling axis - with a result being to restore or enhance T-cell function (e.g., proliferation, cytokine production, and/or target cell killing). As used herein, a PD-1 axis binding antagonist includes a PD-1 binding antagonist, a PD-L1 binding antagonist, and a PD-L2 binding antagonist.

The term "PD-1 binding antagonist" refers to a molecule that decreases, blocks, inhibits, abrogates or interferes with signal transduction resulting from the interaction of PD-1 with one or more of its binding partners, such as PD-L1 and/or PD-L2. In some embodiments, the PD-1 binding antagonist is a molecule that inhibits the binding of PD-1 to one or more of its binding partners. In a specific aspect, the PD-1 binding antagonist inhibits the binding of PD-1 to PD-L1 and/or PD-L2. For example, PD-1 binding antagonists include anti-PD-1 antibodies, antigen-binding fragments thereof, immunoadhesins, fusion proteins, oligopeptides, and other molecules that decrease, block, inhibit, abrogate or interfere with signal transduction resulting from the interaction of PD-1 with PD-L1 and/or PD-L2. In one embodiment, a PD-1 binding antagonist reduces the negative co-stimulatory signal mediated by or through cell surface proteins expressed on T lymphocytes mediated signaling through PD-1 so as render a dysfunctional T-cell less dysfunctional (e.g., enhancing effector responses to antigen recognition). In some embodiments, the PD-1 binding antagonist is an anti-PD-1 antibody. In a specific aspect, a PD-1 binding antagonist is MDX-1106 (nivolumab) described herein. In another specific aspect, a PD-1 binding antagonist is MK-3475 (pembrolizumab) described herein. In another specific aspect, a PD-1 binding antagonist is CT-011 (pidilizumab) described herein. In another specific aspect, a PD-1 binding antagonist is MEDI-0680 (AMP-514) described herein. In another specific aspect, a PD-1 binding antagonist is PDR001 described herein. In another specific aspect, a PD-1 binding antagonist is REGN2810 described herein. In another specific aspect, a PD-1 binding antagonist is BGB-108 described herein.

The term "PD-L1 binding antagonist" refers to a molecule that decreases, blocks, inhibits, abrogates or interferes with signal transduction resulting from the interaction of PD-L1 with either one or more of its binding partners, such as PD-1 and/or B7-1. In some embodiments, a PD-L1 binding antagonist is a molecule that inhibits the binding of PD-L1 to its binding partners. In a specific aspect, the PD-L1 binding antagonist inhibits binding of PD-L1 to PD-1 and/or B7-1. In some embodiments, the PD L1 binding antagonists include anti-PD-Ll antibodies, antigen-binding fragments thereof, immunoadhesins, fusion proteins, oligopeptides and other molecules that decrease, block, inhibit, abrogate or interfere with signal transduction resulting from the interaction of PD-L1 with one or more of its binding partners, such as PD-1 and/or B7-1. In one embodiment, a PD-L1 binding antagonist reduces the negative co-stimulatory signal mediated by or through cell surface proteins expressed on T lymphocytes mediated signaling through PD-L1 so as to render a dysfunctional T-cell less dysfunctional (e.g., enhancing effector responses to antigen recognition). In some embodiments, a PD-L1 binding antagonist is an anti-PD-Ll antibody. According to the present invention, the anti-PD-Ll antibody is MPDL3280A (atezolizumab). In another specific aspect, an anti-PD-Ll antibody is MDX-1105 described herein. In still another specific aspect, an anti-PD-Ll antibody is YW243.55.S70 described herein. In still another specific aspect, an anti-PD-Ll antibody is MEDI4736 (durvalumab) described herein. In still another specific aspect, an anti-PD-Ll antibody is MSB0010718C (avelumab) described herein.

The term "PD-L2 binding antagonist" refers to a molecule that decreases, blocks, inhibits, abrogates or interferes with signal transduction resulting from the interaction of PD-L2 with either one or more of its binding partners, such as PD-1. In some embodiments, a PD-L2 binding antagonist is a molecule that inhibits the binding of PD-L2 to one or more of its binding partners. In a specific aspect, the PD-L2 binding antagonist inhibits binding of PD-L2 to PD-1. In some embodiments, the PD-L2 antagonists include anti-PD-L2 antibodies, antigen binding fragments thereof, immunoadhesins, fusion proteins, oligopeptides and other molecules that decrease, block, inhibit, abrogate or interfere with signal transduction resulting from the interaction of PD-L2 with either one or more of its binding partners, such as PD-1. In one embodiment, a PD-L2 binding antagonist reduces the negative co-stimulatory signal mediated by or through cell surface proteins expressed on T lymphocytes mediated signaling through PD L2 so as render a dysfunctional T-cell less dysfunctional (e.g., enhancing effector responses to antigen recognition). In some embodiments, a PD-L2 binding antagonist is an immunoadhesin.

The term "small molecule" refers to any molecule with a molecular weight of about 2000 daltons or less, preferably of about 500 daltons or less.

"Individual response" or "response" can be assessed using any endpoint indicating a benefit to the individual, including, without limitation, (1) inhibition, to some extent, of disease progression (e.g., cancer progression), including slowing down and complete arrest; (2) a reduction in tumor size; (3) inhibition (*i.e.,* reduction, slowing down or complete stopping) of cancer cell infiltration into adjacent peripheral organs and/or tissues; (4) inhibition (*i.e.* reduction, slowing down or complete stopping) of metasisis; (5) relief, to some extent, of one or more symptoms associated with the disease or disorder (e.g., cancer); (6) increase in the length of progression free survival; and/or (9) decreased mortality at a given point of time following treatment.

An "effective amount" of a substance/molecule, e.g., pharmaceutical composition, refers to an amount effective, at dosages and for periods of time necessary, to achieve the desired therapeutic or prophylactic result.

A "therapeutically effective amount" of a substance/molecule may vary according to factors such as the disease state, age, sex, and weight of the individual, and the ability of the substance/molecule to elicit a desired response in the individual. A therapeutically effective amount is also one in which any toxic or detrimental effects of the substance/molecule are outweighed by the therapeutically beneficial effects. A "prophylactically effective amount" refers to an amount effective, at dosages and for periods of time necessary, to achieve the desired prophylactic result. Typically but not necessarily, since a prophylactic dose is used in subjects prior to or at an earlier stage of disease, the prophylactically effective amount will be less than the therapeutically effective amount.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject., A pharmaceutically acceptable carrier includes, but is not limited to, a buffer, excipient, stabilizer, or preservative.

The phrase "pharmaceutically acceptable salt" as used herein, refers to pharmaceutically acceptable organic or inorganic salts of a compound.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies of the invention are used to delay development of a disease or to slow the progression of a disease.

An "individual" or "subject" is a mammal. Mammals include, but are not limited to, domesticated animals (*e.g.,* cows, sheep, cats, dogs, and horses), primates (*e.g.,* humans and non-human primates such as monkeys), rabbits, and rodents (e.g., mice and rats). In certain embodiments, the individual or subject is a human.

The term "concomitantly" is used herein to refer to administration of two or more therapeutic agents, give in close enough temporal proximity where their individual therapeutic effects overlap in time. In some embodiments, the concomitantly administration is concurrently, sequentially, and/or simultaneously. In some embodiments, concurrent administration includes a dosing regimen when the administration of one or more agent(s) continues after discontinuing the administration of one or more other agent(s).

By "reduce or inhibit" is meant the ability to cause an overall decrease of 20%, 30%, 40%, 50%, 60%, 70%, 75%, 80%, 85%, 90%, 95%, or greater. Reduce or inhibit can refer to the symptoms of the disorder being treated, the presence or size of metastases, or the size of the primary tumor.

The term "package insert" is used to refer to instructions customarily included in commercial packages of therapeutic products, that contain information about the indications, usage, dosage, administration, combination therapy, contraindications and/or warnings concerning the use of such therapeutic products.

An "article of manufacture" is any manufacture (e.g., a package or container) or kit comprising at least one reagent, *e.g.,* a medicament for treatment of a disease or disorder (*e.g.,* cancer), or a probe for specifically detecting a biomarker described herein. In certain embodiments, the manufacture or kit is promoted, distributed, or sold as a unit for performing the methods described herein.

As is understood by one skilled in the art, reference to "about" a value or parameter herein includes (and describes) embodiments that are directed to that value or parameter per se. For example, description referring to "about X" includes description of "X".

It is understood that aspect and embodiments of the invention described herein include "consisting" and/or "consisting essentially of' aspects and embodiments. As used herein, the singular form "a", "an", and "the" includes plural references unless indicated otherwise.

### II. Methods and Uses

Provided herein are methods utilizing a B-RAF inhibitor (e.g., vemurafenib) and a cancer immune checkpoint inhibitor (e.g., an anti-PD1/anti-PD-L1 antibody) for treating cancer. In addition, provided herein are methods utilizing a B-RAF inhibitor *(e.g.,* vemurafenib), a MEK inhibitor *(e.g.,* cobimetinib) and an immune checkpoint inhibitor (e.g., a PD1 axis inhibitor) for treating cancer.

Provided herein are methods of treating cancer in an individual comprising first administering to the individual an effective amount of a B-RAF inhibitor and second administering to the individual an effective amount of the B-RAF inhibitor and an effective amount of an immune checkpoint inhibitor (e.g., a PD1 axis inhibitor). For example, provided herein are methods of treating cancer in an individual comprising first administering to the individual an effective amount of vemurafenib and second administering to the individual an effective amount of vemurafenib and an effective amount of an anti-PD1/anti-PD-L1 antibody (e.g., atezolizumab).

Also provided herein are methods of treating cancer in an individual comprising first administering to the individual an effective amount of a B-RAF inhibitor and an effective amount of a MEK inhibitor, and second administering to the individual an effective amount of the B-RAF inhibitor, an effective amount of the MEK inhibitor, and an effective amount of an immune checkpoint inhibitor (e.g., a PD1 axis inhibitor). For example, provided herein are methods of treating cancer in an individual comprising first administering to the individual an effective amount of vemurafenib and an effective amount of cobimetinib and second administering to the individual an effective amount of vemurafenib, an effective amount of cobimetinib, and an effective amount of an anti-PD1/anti-PD-L1 antibody (e.g., atezolizumab).

Provided herein are methods of increasing efficacy of a cancer treatment comprising first administering to the individual an effective amount of a B-RAF inhibitor and second administering to the individual an effective amount of the B-RAF inhibitor and an effective amount of an immune checkpoint inhibitor (e.g., a PD1 axis inhibitor). For example, provided herein are methods of increasing efficacy of a cancer treatment comprising first administering to the individual an effective amount of vemurafenib and second administering to the individual an effective amount of vemurafenib and an effective amount of an anti-PD1/anti-PD-L1 antibody (e.g., atezolizumab). In some embodiments, the method increase immune tumor CD8+ T-cell infiltration. In some embodiments, the method increases PD-L1 expression, for example, as determined by IHC.

Further, provided herein are methods of increasing efficacy of a cancer treatment comprising first administering to the individual an effective amount of a B-RAF inhibitor and an effective amount of a MEK inhibitor, and second administering to the individual an effective amount of the B-RAF inhibitor, an effective amount of the MEK inhibitor, and an effective amount of an immune checkpoint inhibitor (*e.g*., a PD1 axis inhibitor). For example, provided herein are methods of increasing efficacy of a cancer treatment comprising first administering to the individual an effective amount of vemurafenib and an effective amount of cobimetinib and second administering to the individual an effective amount of vemurafenib, an effective amount of cobimetinib, and an effective amount of an anti-PD1/anti-PD-L1 antibody (e.g., atezolizumab).

Also provided herein are methods of treating cancer in an individual wherein cancer treatment comprises first administering to the individual an effective amount of a B-RAF inhibitor and second administering to the individual an effective amount of the B-RAF inhibitor and an effective amount of an immune checkpoint inhibitor(e.g., a PD1 axis inhibitor), wherein the cancer treatment has increased efficacy compared to administering to the individual an effective amount of the B-RAF inhibitor and an effective amount of immune checkpoint inhibitor (e.g., a PD1 axis inhibitor) alone

(solely concurrent administration). For example, provided herein are methods of treating cancer in an individual wherein cancer treatment comprises first administering to the individual an effective amount of vemurafenib and second administering to the individual an effective amount of vemurafenib and an effective amount of an anti-PD1/anti-PD-L1 antibody (e.g., atezolizumab), wherein the cancer treatment has increased efficacy compared to administering to the individual an effective amount of vemurafenib and an effective amount of the anti-PD1/anti-PD-L1 antibody (e.g., atezolizumab) alone (solely concurrent administration).

Provided herein are methods of treating cancer in an individual wherein cancer treatment comprises first administering to the individual an effective amount of a B-RAF inhibitor and an effective amount of a MEK inhibitor, and second administering to the individual an effective amount of the B-RAF inhibitor, an effective amount of the MEK inhibitor, and an effective amount of an immune checkpoint inhibitor (*e.g.*, a PD1 axis inhibitor), wherein the cancer treatment has increased efficacy compared to administering to the individual an effective amount of the B-RAF inhibitor, an effective amount of the MEK inhibitor, and an effective amount of immune checkpoint inhibitor (e.g., a PD1 axis inhibitor) alone (solely concurrent administration). For example, provided herein are methods of treating cancer in an individual wherein cancer treatment comprises first administering to the individual an effective amount of vemurafenib and an effective amount of cobimetinib and second administering to the individual an effective amount of vemurafenib, an effective amount of cobimetinib and an effective amount of an anti-PD1/anti-PD-L1 antibody (e.g., atezolizumab), wherein the cancer treatment has increased efficacy compared to administering to the individual an effective amount of vemurafenib, effective amount of cobimetinib, and an effective amount of the anti-PD1/anti-PD-L1 antibody (e.g., atezolizumab) alone (solely concurrent administration).

In some embodiments of any of the methods, the first administration is about any of 7, 14, 21, 28, 35, 42, 49, or 56 days. In some embodiments, the first administration is between about 21 and 35 days. In some embodiments, the first administration is about 28 days. In some embodiments, the first administration is about 56 days. For example, methods of treating cancer in an individual comprising first administering to the individual an effective amount of vemurafenib for 28 days and second administering to the individual an effective amount of vemurafenib and an effective amount of an anti-PD1/anti-PD-L1 antibody (*e.g*., atezolizumab). An example of the present invention are methods of treating cancer in an individual comprising first administering to the individual an effective amount of vemurafenib and an effective amount of cobimetinib for 28 days and second administering to the individual an effective amount of vemurafenib, an effective amount of cobimetinib, and an effective amount of an anti-PD1/anti-PD-L1 antibody (atezolizumab).

In some embodiments of any of the methods, the individual according to any of the above embodiments may be a human.

In some embodiments of any of the methods, the first administration comprises administration of a single dosage of the B-RAF inhibitor and/or the MEK inhibitor. In some embodiments of any of the methods, the first administration comprises administration of more than one dosages of the B-RAF inhibitor and/or the MEK inhibitor. In some embodiments, the first administration comprising administering a first dosage for about any of 7, 14, 21, 28, 35, 42, or 49 days followed by a second dosage for about any of 7, 14, 21, 28, 35, 42, or 49 days. In some embodiments, the first administration comprising administering an first dosage for about 49 days followed by a second dosage for about any of 7 days. In some embodiments, the first administration comprising administering an first dosage for about 21 days followed by a second dosage for about any of 7 days. In some embodiments of any of the methods, the first administration comprises a first dosage of the B-RAF inhibitor and/or the MEK inhibitor which is greater than second dosages. For example, methods of treating cancer in an individual comprising first administering to the individual an effective amount of vemurafenib, wherein vemurafenib is administered at a first dosage for 21 days followed by a second dosage for 7 days, and second administering to the individual an effective amount of vemurafenib and an effective amount of an anti-PD1/anti-PD-L1 antibody (e.g., atezolizumab). Also, for example, methods of treating cancer in an individual comprising first administering to the individual an effective amount of vemurafenib and an effective amount of cobimetinib, wherein vemurafenib is administered at a first dosage for 21 days followed by a second dosage for 7 days, and second administering to the individual an effective amount of vemurafenib, an effective amount of cobimetinib, and an effective amount of an anti-PD1/anti-PD-L1 antibody (e.g., atezolizumab).

In some embodiments of any of the methods, the combination therapies noted above encompass combined administration (where two or more therapeutic agents are included in the same or separate formulations), and separate administration, in which case, administration of the antagonist of the invention can occur prior to, simultaneously, sequentially, concurrently and/or following, administration of the additional therapeutic agent and/or adjuvant. In some embodiments, the combination therapy further comprises radiation therapy and/or additional therapeutic agents.

B-RAF inhibitors, MEK inhibitors and/or immune checkpoint inhibitors described herein can be administered by any suitable means, including oral, parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, e.g., by injections, such as intravenous or subcutaneous injections, depending in part on whether the administration is brief or chronic. Various dosing schedules including but not limited to single or multiple administrations over various time-points, bolus administration, and pulse infusion are contemplated herein.

In some embodiments of any of the methods, the administration of the effective amount of the B-RAF, the effective amount of the MEK inhibitor, and/or the effective amount of the immune checkpoint inhibitor may occur via different routes of administration and/or different times of administration. For example, the B-RAF inhibitor may be administered orally, e.g., orally twice a day. The MEK inhibitor may be administered orally, e.g., orally once a day. In some embodiments, when the first administration includes a B-RAF inhibitor and a MEK inhibitor, the B-RAF inhibitor and the MEK inhibitor act concomitantly. In some embodiments, when the second administration includes a B-RAF inhibitor and a immune checkpoint inhibitor, the B-RAF inhibitor and the immune checkpoint inhibitor act concomitantly. In some embodiments, when the second administration includes a B-RAF inhibitor, a MEK inhibitor, and an immune checkpoint inhibitor, the B-RAF inhibitor, the MEK inhibitor, and the immune checkpoint inhibitor act concomitantly. In some embodiments, the first and second administrations act concomitantly. In some embodiments, the first administration and second administration are sequential.

B-RAF inhibitors, MEK inhibitors and/or immune checkpoint inhibitors described herein may be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The B-RAF inhibitors, MEK inhibitors and/or immune checkpoint inhibitors need not be, but is optionally formulated with one or more agents currently used to prevent or treat the disorder in question. The effective amount of such other agents depends on the amount of the B-RAF inhibitors, MEK inhibitors and/or immune checkpoint inhibitors present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

For the prevention or treatment of disease, the appropriate dosage of the B-RAF inhibitor, MEK inhibitor and/or immune checkpoint inhibitor described herein (when used alone or in combination with one or more other additional therapeutic agents) will depend on the type of disease to be treated, the severity and course of the disease, whether the the B-RAF inhibitor, MEK inhibitor and/or immune checkpoint inhibitor is administered for preventive or therapeutic purposes, previous therapy, the patient's clinical history and response to the B-RAF inhibitor, MEK inhibitor and/or immune checkpoint inhibitor and the discretion of the attending physician. The B-RAF inhibitor, MEK inhibitor and/or immune checkpoint inhibitor is suitably administered to the patient at one time or over a series of treatments. For repeated administrations over several days or longer, depending on the condition, the treatment would generally be sustained until a desired suppression of disease symptoms occurs.

In some embodiments, the B-RAF inhibitor (*e.g*., vemurafenib) is administered daily or twice a day. In some embodiments, the B-RAF inhibitor (*e.g*., vemurafenib) is administered twice a day at a dosage of about any of 960 mg, 720 mg, 480 mg, or 240 mg. In some embodiments, the first administration comprises administering the B-RAF inhibitor (*e.g*., vemurafenib) at a first dosage of 960 mg twice a day followed a second dosage of 720 mg twice a day. In some embodiments, the first administration comprises administering the B-RAF inhibitor (*e.g*., vemurafenib) at a first dosage of 720 mg twice a day followed a second dosage of 480 mg twice a day. In some embodiments, the second administration comprising administering the B-RAF inhibitor (*e.g*., vemurafenib) at a dosage of 960 mg twice a day. In some embodiments, the second administration comprising administering the B-RAF inhibitor (*e.g*., vemurafenib) at a dosage of 720 mg twice a day. For example, in some embodiments, methods of treating cancer in an individual comprising first administering to the individual a B-RAF inhibitor (*e.g.,* vemurafenib) for 28 days, wherein B-RAF inhibitor (*e.g.,* vemurafenib) is administered at 960 mg twice a day for 21 days and 720 mg twice a day for 7 days and second administering to the individual a B-RAF inhibitor (*e.g*., vemurafenib) at a dosage of 720 mg twice a day and an effective amount of an anti-PD1/anti-PD-L1 antibody (*e.g*., atezolizumab).

In some embodiments, the MEK inhibitor (*e.g*., cobimetinib) is administered daily or twice a day. In some embodiments, the MEK inhibitor (*e.g*., cobimetinib) is administered daily at a dosage of about any of 60 mg or 20 mg. In some embodiments, the first administration comprises administering the MEK inhibitor (*e.g*., cobimetinib) at a first dosage of 60 mg daily for 21 days on and 7 days off (21/7). In some embodiments, the first administration comprises administering the MEK inhibitor (*e.g*., cobimetinib) at a first dosage of 40 mg daily for 21 days on and 7 days off (21/7). In some embodiments, the second administration comprising administering the MEK inhibitor (*e.g*., cobimetinib) at a dosage of 60 mg daily for 21 days on and 7 days off (21/7). In some embodiments, the second administration comprising administering the MEK inhibitor (*e.g*., cobimetinib) at a dosage of 40 mg daily for 21 days on and 7 days off (21/7). For example, in some embodiments, methods of treating cancer in an individual comprising first administering to the individual a B-RAF inhibitor (*e.g.,* vemurafenib) and MEK inhibitor (*e.g.,* cobimetinib) for 28 days, wherein B-RAF inhibitor (*e.g.,* vemurafenib) is administered at 960 mg twice a day for 21 days and 720 mg twice a day for 7 days and MEK inhibitor (*e.g*., cobimetinib) is administered at a dosage of 60 mg daily for 21 days on and 7 days off and second administering to the individual a B-RAF inhibitor (*e.g*., vemurafenib) at a dosage of 720 mg twice a day, MEK inhibitor *(e.g.,* cobimetinib) at a dosage of 60 mg daily for 21 days on and 7 days off and an effective amount of an anti-PD1/anti-PD-L1 antibody (*e.g*., atezolizumab).

In some embodiments, the anti-PD1/anti-PD-L1 antibody (*e.g*., atezolizumab) is administered every 3 weeks (q3w) or every two weeks (q2w). In some embodiments, the anti-PD1/anti-PD-L1 antibody (*e.g*., atezolizumab) is administered daily at a dosage of about 15 mg/kg q3w, about 20 mg/kg q3w, about 800 mg q2w, or about 1200 mg q3w. In some embodiments, the second administration comprising administering the anti-PD1/anti-PD-L1 antibody (*e.g*., atezolizumab) at a dosage of 800 mg q2w. In some embodiments, the second administration comprising administering the anti-PD1/anti-PD-L1 antibody (*e.g*., atezolizumab) at a dosage of 1200 mg q3w. For example, in some embodiments, methods of treating cancer in an individual comprising first administering to the individual a B-RAF inhibitor (*e.g.,* vemurafenib) for 28 days, wherein B-RAF inhibitor (*e.g.,* vemurafenib) is administered at 960 mg twice a day for 21 days and 720 mg twice a day for 7 days and second administering to the individual a B-RAF inhibitor (*e.g*., vemurafenib) at a dosage of 720 mg twice a day and an anti-PD1/anti-PD-L1 antibody (*e.g*., atezolizumab) at a dosage of 1200 mg q3w.For example, in some embodiments, methods of treating cancer in an individual comprising first administering to the individual a B-RAF inhibitor (*e.g.,* vemurafenib) and MEK inhibitor (*e.g.,* cobimetinib) for 28 days, wherein B-RAF inhibitor (e.g., vemurafenib) is administered at 960 mg twice a day for 21 days and 720 mg twice a day for 7 days and MEK inhibitor *(e.g.,* cobimetinib) is administered at a dosage of 60 mg daily for 21 days on and 7 days off and second administering to the individual a B-RAF inhibitor (*e.g*., vemurafenib) at a dosage of 720 mg twice a day, MEK inhibitor (*e.g*., cobimetinib) at a dosage of 60 mg daily for 21 days on and 7 days off and an anti-PD1/anti-PD-L1 antibody (*e.g*., atezolizumab) at a dosage of 800 mg q2w.

In some embodiments of any of the methods, the cancer is melanoma. In some embodiments, the melanoma is unresectable or metastatic melanoma. In some embodiments, the melanoma is B-RAF V600 mutant melanoma. In some embodiments, the B-RAF V600 mutant melanoma is B-RAF V600E mutant melanoma. In some embodiments, the B-RAF V600 mutant melanoma is B-RAF V600K mutant melanoma.

### III. Therapeutic Compositions

Provided herein are combinations comprising a B-RAF inhibitor, a MEK inhibitor, and an immune checkpoint inhibitor. In certain embodiments, the combination increases the efficacy compared to treatment comprising administering comprising a B-RAF inhibitor, a MEK inhibitor, or a immune checkpoint inhibitor alone or coadministration (concurrent administration) of a B-RAF inhibitor, a MEK inhibitor, or an immune checkpoint inhibitor. In certain embodiments, the combination comprising a B-RAF inhibitor, a MEK inhibitor, and an immune checkpoint inhibitor as described herein increases T-cell infiltration and/or PD-L1 expression levels compared to treatment comprising administering comprising a B-RAF inhibitor, a MEK inhibitor, or a immune checkpoint inhibitor alone or coadministration (concurrent administration) of a B-RAF inhibitor, a MEK inhibitor, or an immune checkpoint inhibitor.

In some embodiments of any of the methods, the methods described herein may use a B-RAF inhibitor. Exemplary BRAF inhibitors are known in the art and include, for example, sorafenib, PLX4720, PLX-3603, dabrafenib (GSK2118436), encorafenib (LGX818), GDC-0879, RAF265 (Novartis), XL281, ARQ736, BAY73-4506, vemurafenib and those described in WO2007/002325, WO2007/002433, WO2009111278, WO2009111279, WO2009111277, WO2009111280 and U.S. Pat. No. 7,491,829. In some embodiments, the BRAF inhibitor is a selective BRAF inhibitor. In some embodiments, the BRAF inhibitor is a selective inhibitor of BRAF V600. In some embodiments, BRAF V600 is BRAF V600E, BRAF V600K, and/or V600D. In some embodiments, BRAF V600 is BRAF V600R. According to the present invention, the B-RAF inhibitor is vemurafenib.

Vemurafenib (RG7204, PLX-4032, CAS Reg. No. 1029872-55-5) has been shown to cause programmed cell death in various cancer call lines, for example melanoma cell lines. Vemurafenib interrupts the BRAF/MEK step on the BRAF/MEK/ERK pathway - if the BRAF has the common V600E mutation. Vemurafenib works in patients, for example in melanoma patients as approved by the FDA, whose cancer has a V600E BRAF mutation (that is, at amino acid position number 600 on the BRAF protein, the normal valine is replaced by glutamic acid). About 60% of melanomas have the V600E BRAF mutation. The V600E mutation is present in a variety of other cancers, including lymphoma, colon cancer, melanoma, thyroid cancer and lung cancer. Vemurafenib has the following structure:

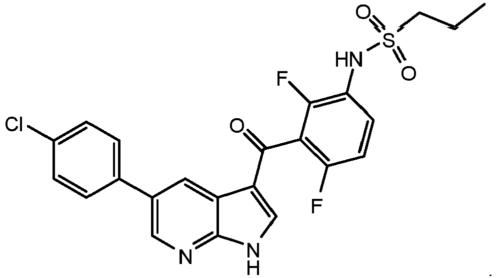

ZELBORAF® (vemurafenib) (Genentech, Inc.) is a drug product approved in the U.S. and indicated for treatment of patients with unresectable or metastatic melanoma with BRAF V600E mutation as detected by an FDA-approved test. ZELBORAF® (vemurafenib) is not recommended for use in melanoma patients who lack the BRAF V600E mutation (wild-type BRAF melanoma). ZELBORAF (vemurafenib) is a kinase inhibitor available as 240 mg tablets for oral use.

In some embodiments of any of the combination therapy methods described herein, the targeted therapeutic is a MEK inhibitor. In some embodiments, the MEK inhibitor is a MEK1 inhibitor, MEK2 inhibitor, and/or MEK1/2 inhibitor. Exemplary MEK inhibitors include, but are not limited to, trametinib (GSK 1120212), MEK162, selumetinib (AZD 6244, ARRY-142886), pimasertib (MSC1936369B, AS-703026, AS703026), refametinib, cobimetinib (GDC-0973), BI-847325, GDC-0623, PD-325901, CI-1040, and PD035901. In some embodiments, the MEK inhibitor is selumetinib, pimasertib, cobimetinib (GDC-0973), GDC-0623, binimetinib or trametinib. According to the present invention, the MEK inhibitor is cobimetinib (GDC-0973).

Cobimetinib (GDC-0973 or XL518) is a selective inhibitor of MEK, also known as mitogen activated protein kinase kinase (MAPKK), which is a key component of the RAS/RAF/MEK/ERK pathway that is frequently activated in human tumors. GDC-0973 can be prepared as described in International Patent Application Publication Number WO2007044515(A1). GDC-0973 has the name: (S)-(3,4-difluoro-2-(2-fluoro-4-iodophenylamino)phenyl)(3-hydroxy-3-(piperidin-2-yl)azetidin-1-yl)methanone, and the following structure: 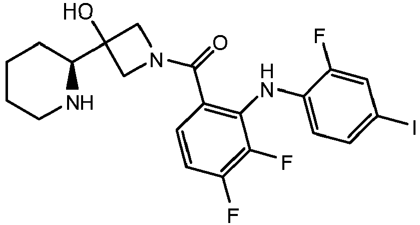 In some embodiments, the MEK inhibitor is (S)-[3,4-difluoro-2-(2-fluoro-4-iodophenylamino)phenyl] [3-hydroxy-3-(piperidin-2-yl)azetidin-1-yl]methanone or a pharmaceutically acceptable salt thereof. In some embodiments, the MEK inhibitor is (S)-[3,4-difluoro-2-(2-fluoro-4-iodophenylamino)phenyl] [3-hydroxy-3-(piperidin-2-yl)azetidin-l-yl]methanone, hemifumarate. In some embodiments, the MEK inhibitor is cobimetinib.

Cobimetinib is a fumarate salt appearing as white to off-white solid and exhibits a pH dependent solubility. COTELLIC (cobimetinib) tablets are supplied as white, round, film-coated 20 mg tablets for oral administration, debossed on one side with "COB". Each 20 mg tablet contains 22 mg of cobimetinib fumarate, which corresponds to 20 mg of the cobimetinib free base. The inactive ingredients of COTELLIC are: Tablet Core: microcrystalline cellulose, lactose monohydrate, croscarmellose sodium, magnesium stearate. Coating: polyvinyl alcohol, titanium dioxide, polyethylene glycol 3350, talc.

In some embodiments of any of the methods, the methods described herein may use a immune checkpoint inhibitor. In some embodiments, the immune check point inhibitor is a PD-1 axis binding antagonist. In some embodiments, a PD-1 axis binding antagonist includes a PD-1 binding antagonist, a PD-L1 binding antagonist and a PD-L2 binding antagonist. PD-1 (programmed death 1) is also referred to in the art as "programmed cell death 1," "PDCD1," "CD279," and "SLEB2." An exemplary human PD-1 is shown in UniProtKB/Swiss-Prot Accession No. Q15116. PD-L1 (programmed death ligand 1) is also referred to in the art as "programmed cell death 1 ligand 1," "PDCD1LG1," "CD274," "B7-H," and "PDL1." An exemplary human PD-L1 is shown in UniProtKB/Swiss-Prot Accession No.Q9NZQ7.1. PD-L2 (programmed death ligand 2) is also referred to in the art as "programmed cell death 1 ligand 2," "PDCD1LG2," "CD273," "B7-DC," "Btdc," and "PDL2." An exemplary human PD-L2 is shown in UniProtKB/Swiss-Prot Accession No. Q9BQ51. In some embodiments, PD-1, PD-L1, and PD-L2 are human PD-1, PD-L1 and PD-L2.

In some embodiments, the PD-1 binding antagonist is a molecule that inhibits the binding of PD-1 to its ligand binding partners. In a specific aspect the PD-1 ligand binding partners are PD-L1 and/or PD-L2. In another embodiment, a PD-L1 binding antagonist is a molecule that inhibits the binding of PD-L1 to its binding partners. In a specific aspect, PD-L1 binding partners are PD-1 and/or B7-1. In another embodiment, the PD-L2 binding antagonist is a molecule that inhibits the binding of PD-L2 to its binding partners. In a specific aspect, a PD-L2 binding partner is PD-1. The antagonist may be an antibody, an antigen binding fragment thereof, an immunoadhesin, a fusion protein, or oligopeptide.

In some embodiments, the PD-1 binding antagonist is an anti-PD-1 antibody (e.g., a human antibody, a humanized antibody, or a chimeric antibody). In some embodiments, the anti-PD-1 antibody is selected from the group consisting of MDX 1106 (nivolumab), MK-3475 (pembrolizumab), CT-011 (pidilizumab), MEDI-0680 (AMP-514), PDR001, REGN2810, and BGB-108. In some embodiments, the PD-1 binding antagonist is an immunoadhesin (e.g., an immunoadhesin comprising an extracellular or PD-1 binding portion of PD-L1 or PD-L2 fused to a constant region *(e.g.,* an Fc region of an immunoglobulin sequence). In some embodiments, the PD-1 binding antagonist is AMP-224. In some embodiments, the PD-L1 binding antagonist is anti-PD-Ll antibody. In some embodiments, the anti-PD-Ll antibody is selected from the group consisting of MPDL3280A, YW243.55.S70, MDX-1105, MEDI4736 (durvalumab), and MSB0010718C (avelumab). Antibody YW243.55.S70 is an anti-PD-Ll described in WO 2010/077634. MDX-1105, also known as BMS-936559, is an anti-PD-Ll antibody described in WO2007/005874. MEDI4736 is an anti-PD-Ll monoclonal antibody described in WO2011/066389 and US2013/034559. MDX-1106, also known as MDX-1106-04, ONO-4538, BMS-936558, or nivolumab, is an anti-PD-1 antibody described in WO2006/121168. MK-3475, also known as lambrolizumab, is an anti-PD-1 antibody described in WO2009/114335. CT-011, also known as hBAT, hBAT-1 or pidilizumab, is an anti-PD-1 antibody described in WO2009/101611. AMP-224, also known as B7-DCIg, is a PD-L2-Fc fusion soluble receptor described in WO2010/027827 and WO2011/066342.

In some embodiments, the PD-1 axis binding antagonist is an anti-PD-Ll antibody. In some embodiments, the anti-PD-Ll antibody is capable of inhibiting binding between PD-L1 and PD-1 and/or between PD-L1 and B7-1. In some embodiments, the anti-PD-Ll antibody is a monoclonal antibody. In some embodiments, the anti-PD-Ll antibody is an antibody fragment selected from the group consisting of Fab, Fab'-SH, Fv, scFv, and (Fab')2 fragments. In some embodiments, the anti-PD-L1 antibody is a humanized antibody. In some embodiments, the anti-PD-Ll antibody is a human antibody.

Examples of anti-PD-Ll antibodies useful for the methods herein are described in PCT patent application WO 2010/077634, WO 2007/005874, WO 2011/066389, and US 2013/0345 59.

### Anti-PD-1 antibodies

In some embodiments, the anti-PD-1 antibody is MDX-1106. Alternative names for "MDX-1106" include MDX-1106-04, ONO-4538, BMS-936558, or nivolumab. In some embodiments, the anti-PD-1 antibody is nivolumab (CAS Registry Number: 946414-94-4). In a still further embodiment, provided is an isolated anti-PD-1 antibody comprising a heavy chain variable region comprising the heavy chain variable region amino acid sequence from SEQ ID NO: 1 and/or a light chain variable region comprising the light chain variable region amino acid sequence from SEQ ID NO:2. In a still further embodiment, provided is an isolated anti-PD-1 antibody comprising a heavy chain and/or a light chain sequence, wherein:
(a) the heavy chain sequence has at least 85%, at least 90%, at least 91%, at least 92%, at least 93%, at least 94%, at least 95%, at least 96%, at least 97%, at least 98%, at least 99% or 100% sequence identity to the heavy chain sequence: 
QVQLVESGGGVVQPGRSLRLDCKASGITFSNSGMHWVRQAPGKGLEWVAVIWYDGSKRYY
ADSVKGRFTISRDNSKNTLFLQMNSLRAEDTAVYYCATNDDYWGQGTLVTVSSASTKGPSVF
PLAPCSRSTSESTAALGCLVKDYFPEPVTVSWNSGALTSGVHTFPAVLQSSGLYSLSSVVTVPS
SSLGTKTYTCNVDHKPSNTKVDKRVESKYGPPCPPCPAPEFLGGPSVFLFPPKPKDTLMISRTP
EVTCVVVDVSQEDPEVQFNWYVDGVEVHNAKTKPREEQFNSTYRVVSVLTVLHQDWLNGK
EYKCKVSNKGLPSSIEKTISKAKGQPREPQVYTLPPSQEEMTKNQVSLTCLVKGFYPSDIAVE
WESNGQPENNYKTTPPVLDSDGSFFLYSRLTVDKSRWQEGNVFSCSVMHEALHNHYTQKSLS
LSLGK (SEQ ID NO:1),
 and
(b) the light chain sequences has at least 85%, at least 90%, at least 91%, at least 92%, at least 93%, at least 94%, at least 95%, at least 96%, at least 97%, at least 98%, at least 99% or 100% sequence identity to the light chain sequence:

EIVLTQSPATLSLSPGERATLSCRASQSVSSYLAWYQQKPGQAPRLLIYDASNRATGIPARFSG
SGSGTDFTLTISSLEPEDFAVYYCQQSSNWPRTFGQGTKVEIKRTVAAPSVFIFPPSDEQLKSGT
ASVVCLLNNFYPREAKVQWKVDNALQSGNSQESVTEQDSKDSTYSLSSTLTLSKADYEKHK
VYACEVTHQGLSSPVTKSFNRGEC (SEQ ID NO:2).

### Anti-PD-L1 antibodies

In some embodiments, the antibody in the formulation comprises at least one tryptophan (e.g., at least two, at least three, or at least four) in the heavy and/or light chain sequence. In some embodiments, amino acid tryptophan is in the HVR regions, framework regions and/or constant regions of the antibody. In some embodiments, the antibody comprises two or three tryptophan residues in the HVR regions. In some embodiments, the antibody in the formulation is an anti-PD-Ll antibody. PD-L1 (programmed death ligand 1), also known as PDL1, B7-H1, B7-4, CD274, and B7-H, is a transmembrane protein, and its interaction with PD-1 inhibits T-cell activation and cytokine production. In some embodiments, the anti-PD-Ll antibody described herein binds to human PD-L1. Examples of anti-PD-Ll antibodies that can be used in the methods described herein are described in PCT patent application WO 2010/077634 A1 and U.S. Patent No. 8,217,1 49.

In some embodiments, the anti-PD-Ll antibody is capable of inhibiting binding between PD-L1 and PD-1 and/or between PD-L1 and B7-1. In some embodiments, the anti-PD-Ll antibody is a monoclonal antibody. In some embodiments, the anti-PD-Ll antibody is an antibody fragment selected from the group consisting of Fab, Fab'-SH, Fv, scFv, and (Fab')₂ fragments. In some embodiments, the anti-PD-Ll antibody is a humanized antibody. In some embodiments, the anti-PD-L1 antibody is a human antibody.

Anti-PD-Ll antibodies described in WO 2010/077634 A1 and US 8,217,149 may be used in the methods described herein. In some embodiments, the anti-PD-Ll antibody comprises a heavy chain variable region sequence of SEQ ID NO:3 and/or a light chain variable region sequence of SEQ ID NO:4. In a still further embodiment, provided is an isolated anti-PD-Ll antibody comprising a heavy chain variable region and/or a light chain variable region sequence, wherein:
(a) the heavy chain sequence has at least 85%, at least 90%, at least 91%, at least 92%, at least 93%, at least 94%, at least 95%, at least 96%, at least 97%, at least 98%, at least 99% or 100% sequence identity to the heavy chain sequence: 
EVQLVESGGGLVQPGGSLRLSCAASGFTFSDSWIHWVRQAPGKGLEWVAWISPYGGSTYYA
DSVKGRFTISADTSKNTAYLQMNSLRAEDTAVYYCARRHWPGGFDYWGQGTLVTVSA (SEQ
ID NO:3),
 and
(b) the light chain sequence has at least 85%, at least 90%, at least 91%, at least 92%, at least 93%, at least 94%, at least 95%, at least 96%, at least 97%, at least 98%, at least 99% or 100% sequence identity to the light chain sequence:

DIQMTQSPSSLSASVGDRVTITCRASQDVSTAVAWYQQKPGKAPKLLIYSASFLYSGVPSRFSG
SGSGTDFTLTISSLQPEDFATYYCQQYLYHPATFGQGTKVEIKR (SEQ ID NO:4).

In one embodiment, the anti-PD-Ll antibody comprises a heavy chain variable region comprising an HVR-H1, HVR-H2 and HVR-H3 sequence, wherein:

| | |
|---|---|
| (a) the HVR-H1 sequence is GFTFSX₁SWIH | (SEQ ID NO: 5); |
| (b) the HVR-H2 sequence is AWIX₂PYGGSX₃YYADSVKG | (SEQ ID NO:6); |
| (c) the HVR-H3 sequence is RHWPGGFDY | (SEQ ID NO:7); |

further wherein: X₁ is D or G; X₂ is S or L; X₃ is T or S. In one specific aspect, X₁ is D; X₂ is S and X₃ is T.

In another aspect, the polypeptide further comprises variable region heavy chain framework sequences juxtaposed between the HVRs according to the formula: (HC-FR1)-(HVR-H1)-(HC-FR2)-(HVR-H2)-(HC-FR3)-(HVR-H3)-(HC-FR4). In yet another aspect, the framework sequences are derived from human consensus framework sequences. In a further aspect, the framework sequences are VH subgroup III consensus framework. In a still further aspect, at least one of the framework sequences is the following:

| | |
|---|---|
| HC-FR1 is EVQLVESGGGLVQPGGSLRLSCAAS | (SEQ ID NO: 8) |
| HC-FR2 is WVRQAPGKGLEWV | (SEQ ID NO:9) |
| HC-FR3 is RFTISADTSKNT A YLQMNSLRAEDT A VYYCAR | (SEQ ID NO: 10) |
| HC-FR4 is WGQGTLVTVSA | (SEQ ID NO: 11). |

In a still further aspect, the heavy chain polypeptide is further combined with a variable region light chain comprising an HVR-L1, HVR-L2 and HVR-L3, wherein:

| | |
|---|---|
| (a) the HVR-L1 sequence is RASQX₄X₅X₆TX₇X₈A | (SEQ ID NO:12); |
| (b) the HVR-L2 sequence is SASX₉LX₁₀S, | (SEQ ID NO:13); |
| (c) the HVR-L3 sequence is QQX₁₁X₁₂X₁₃X₁₄PX₁₅T | (SEQ ID NO:14); |

wherein: X₄ is D or V; X₅ is V or I; X₆ is S or N; X₇ is A or F; X₈ is V or L; X₉ is F or T; X₁₀ is Y or A; X₁₁ is Y, G, F, or S; X₁₂ is L, Y, F or W; X₁₃ is Y, N, A, T, G, F or I; X₁₄ is H, V, P, T or I; X₁₅ is A, W, R, P or T. In a still further aspect, X₄ is D; X₅ is V; X₆ is S; X₇ is A; X₈ is V; X₉ is F; X₁₀ is Y; X₁₁ is Y; X₁₂ is L; X₁₃ is Y; X₁₄ is H; X₁₅ is A.

In a still further aspect, the light chain further comprises variable region light chain framework sequences juxtaposed between the HVRs according to the formula: (LC-FR1)-(HVR-L1)-(LC-FR2)-(HVR-L2)-(LC-FR3)-(HVR-L3)-(LC-FR4). In a still further aspect, the framework sequences are derived from human consensus framework sequences. In a still further aspect, the framework sequences are VL kappa I consensus framework. In a still further aspect, at least one of the framework sequence is the following:

| | |
|---|---|
| LC-FR1 is DIQMTQSPSSLSASVGDRVTITC | (SEQ ID NO:15) |
| LC-FR2 is WYQQKPGKAPKLLIY | (SEQ ID NO:16) |
| LC-FR3 is GVPSRFSGSGSGTDFTLTISSLQPEDFATYYC | (SEQ ID NO:17) |
| LC-FR4 is FGQGTKVEIKR | (SEQ ID NO:18) |

In another embodiment, provided is an isolated anti-PD-Ll antibody or antigen binding fragment comprising a heavy chain and a light chain variable region sequence, wherein:
(a) the heavy chain comprises an HVR-H1, HVR-H2 and HVR-H3, wherein further:

| | |
|---|---|
| (i) the HVR-H1 sequence is GFTFSX₁SWIH; | (SEQ ID NO:5) |
| (ii) the HVR-H2 sequence is AWIX₂PYGGSX₃YYADSVKG | (SEQ ID NO:6) |
| (iii) the HVR-H3 sequence is RHWPGGFDY, and | (SEQ ID NO:7) |

(b) the light chain comprises an HVR-L1, HVR-L2 and HVR-L3, wherein further:

| | |
|---|---|
| (i) the HVR-L1 sequence is RASQX₄X₅X₆TX₇X₈A | (SEQ ID NO:12) |
| (ii) the HVR-L2 sequence is SASX₉LX₁₀S; and | (SEQ ID NO:13) |
| (iii) the HVR-L3 sequence is QQX₁₁X₁₂X₁₃X₁₄PX₁₅T; | (SEQ ID NO:14) |

wherein: X₁ is D or G; X₂ is S or L; X₃ is T or S; X₄ is D or V; X₅ is V or I; X₆ is S or N; X₇ is A or F; X₈ is V or L; X₉ is F or T; X₁₀ is Y or A; X₁₁ is Y, G, F, or S; X₁₂ is L, Y, F or W; X₁₃ is Y, N, A, T, G, F or I; X₁₄ is H, V, P, T or I; X₁₅ is A, W, R, P or T. In a specific aspect, X₁ is D; X₂ is S and X₃ is T. In another aspect, X₄ is D; X₅ is V; X₆ is S; X₇ is A; X₈ is V; X₉ is F; X₁₀ is Y; X₁₁ is Y; X₁₂ is L; X₁₃ is Y; X₁₄ is H; X₁₅ is A. In yet another aspect, X₁ is D; X₂ is S and X₃ is T, X₄ is D; X₅ is V; X₆ is S; X₇ is A; X₈ is V; X₉ is F; X₁₀ is Y; X₁₁ is Y; X₁₂ is L; X₁₃ is Y; X₁₄ is H and X₁₅ is A.

In a further aspect, the heavy chain variable region comprises one or more framework sequences juxtaposed between the HVRs as: (HC-FR1)-(HVR-H1)-(HC-FR2)-(HVR-H2)-(HC-FR3)-(HVR-H3)-(HC-FR4), and the light chain variable regions comprises one or more framework sequences juxtaposed between the HVRs as: (LC-FR1)-(HVR-L1)-(LC-FR2)-(HVR-L2)-(LC-FR3)-(HVR-L3)-(LC-FR4). In a still further aspect, the framework sequences are derived from human consensus framework sequences. In a still further aspect, the heavy chain framework sequences are derived from a Kabat subgroup I, II, or III sequence. In a still further aspect, the heavy chain framework sequence is a VH subgroup III consensus framework. In a still further aspect, one or more of the heavy chain framework sequences are set forth as SEQ ID NOs:8, 9, 10 and 11. In a still further aspect, the light chain framework sequences are derived from a Kabat kappa I, II, II or IV subgroup sequence. In a still further aspect, the light chain framework sequences are VL kappa I consensus framework. In a still further aspect, one or more of the light chain framework sequences are set forth as SEQ ID NOs:15, 16, 17 and 18.

In a still further specific aspect, the antibody further comprises a human or murine constant region. In a still further aspect, the human constant region is selected from the group consisting of IgG1, IgG2, IgG2, IgG3, IgG4. In a still further specific aspect, the human constant region is IgG1. In a still further aspect, the murine constant region is selected from the group consisting of IgG1, IgG2A, IgG2B, IgG3. In a still further aspect, the murine constant region if IgG2A. In a still further specific aspect, the antibody has reduced or minimal effector function. In a still further specific aspect the minimal effector function results from an "effector-less Fc mutation" or aglycosylation. In still a further embodiment, the effector-less Fc mutation is an N297A or D265A/N297A substitution in the constant region.

In yet another embodiment, provided is an anti-PD-Ll antibody comprising a heavy chain and a light chain variable region sequence, wherein:
(a) the heavy chain further comprises an HVR-H1, HVR-H2 and an HVR-H3 sequence having at least 85% sequence identity to GFTFSDSWIH (SEQ ID NO:19), AWISPYGGSTYYADSVKG (SEQ ID NO:20) and RHWPGGFDY (SEQ ID NO:21), respectively, or
(b) the light chain further comprises an HVR-L1, HVR-L2 and an HVR-L3 sequence having at least 85% sequence identity to RASQDVSTAVA (SEQ ID NO:22), SASFLYS (SEQ ID NO:23) and QQYLYHPAT (SEQ ID NO:24), respectively.

In a specific aspect, the sequence identity is 86%, 87%, 88%, 89%, 90%, 91%, 92%, 93%, 94%, 95%, 96%, 97%, 98%, 99% or 100%.

In another aspect, the heavy chain variable region comprises one or more framework sequences juxtaposed between the HVRs as: (HC-FR1)-(HVR-H1)-(HC-FR2)-(HVR-H2)-(HC-FR3)-(HVR-H3)-(HC-FR4), and the light chain variable regions comprises one or more framework sequences juxtaposed between the HVRs as: (LC-FR1)-(HVR-L1)-(LC-FR2)-(HVR-L2)-(LC-FR3)-(HVR-L3)-(LC-FR4). In yet another aspect, the framework sequences are derived from human consensus framework sequences. In a still further aspect, the heavy chain framework sequences are derived from a Kabat subgroup I, II, or III sequence. In a still further aspect, the heavy chain framework sequence is a VH subgroup III consensus framework. In a still further aspect, one or more of the heavy chain framework sequences are set forth as SEQ ID NOs:8, 9, 10 and 11. In a still further aspect, the light chain framework sequences are derived from a Kabat kappa I, II, II or IV subgroup sequence. In a still further aspect, the light chain framework sequences are VL kappa I consensus framework. In a still further aspect, one or more of the light chain framework sequences are set forth as SEQ ID NOs:15, 16, 17 and 18.

In a still further specific aspect, the antibody further comprises a human or murine constant region. In a still further aspect, the human constant region is selected from the group consisting of IgG1, IgG2, IgG2, IgG3, IgG4. In a still further specific aspect, the human constant region is IgG1. In a still further aspect, the murine constant region is selected from the group consisting of IgG1, IgG2A, IgG2B, IgG3. In a still further aspect, the murine constant region if IgG2A. In a still further specific aspect, the antibody has reduced or minimal effector function. In a still further specific aspect the minimal effector function results from an "effector-less Fc mutation" or aglycosylation. In still a further embodiment, the effector-less Fc mutation is an N297A or D265A/N297A substitution in the constant region.

In another further embodiment, provided is an isolated anti-PD-Ll antibody comprising a heavy chain and a light chain variable region sequence, wherein:
(a) the heavy chain sequence has at least 85% sequence identity to the heavy chain sequence: 

 (SEQ
ID NO:25),
 and/or
(b) the light chain sequences has at least 85% sequence identity to the light chain sequence:

DIQMTQSPSSLSASVGDRVTITCRASQDVSTAVAWYQQKPGKAPKLLIYSASFLYSGVPSRFSG
SGSGTDFTLTISSLQPEDFATYYCQQYLYHPATFGQGTKVEIKR (SEQ ID NO:4).

In a specific aspect, the sequence identity is 86%, 87%, 88%, 89%, 90%, 91%, 92%, 93%, 94%, 95%, 96%, 97%, 98%, 99% or 100%. In another aspect, the heavy chain variable region comprises one or more framework sequences juxtaposed between the HVRs as: (HC-FR1)-(HVR-H1)-(HC-FR2)-(HVR-H2)-(HC-FR3)-(HVR-H3)-(HC-FR4), and the light chain variable regions comprises one or more framework sequences juxtaposed between the HVRs as: (LC-FR1)-(HVR-L1)-(LC-FR2)-(HVR-L2)-(LC-FR3)-(HVR-L3)-(LC-FR4). In yet another aspect, the framework sequences are derived from human consensus framework sequences. In a further aspect, the heavy chain framework sequences are derived from a Kabat subgroup I, II, or III sequence. In a still further aspect, the heavy chain framework sequence is a VH subgroup III consensus framework. In a still further aspect, one or more of the heavy chain framework sequences are set forth as SEQ ID NOs:8, 9, 10 and WGQGTLVTVSS (SEQ ID NO:27).

In a still further aspect, the light chain framework sequences are derived from a Kabat kappa I, II, II or IV subgroup sequence. In a still further aspect, the light chain framework sequences are VL kappa I consensus framework. In a still further aspect, one or more of the light chain framework sequences are set forth as SEQ ID NOs:15, 16, 17 and 18.

In a still further specific aspect, the antibody further comprises a human or murine constant region. In a still further aspect, the human constant region is selected from the group consisting of IgG1, IgG2, IgG2, IgG3, IgG4. In a still further specific aspect, the human constant region is IgG1. In a still further aspect, the murine constant region is selected from the group consisting of IgG1, IgG2A, IgG2B, IgG3. In a still further aspect, the murine constant region if IgG2A. In a still further specific aspect, the antibody has reduced or minimal effector function. In a still further specific aspect, the minimal effector function results from production in prokaryotic cells. In a still further specific aspect the minimal effector function results from an "effector-less Fc mutation" or aglycosylation. In still a further embodiment, the effector-less Fc mutation is an N297A or D265A/N297A substitution in the constant region.

In a further aspect, the heavy chain variable region comprises one or more framework sequences juxtaposed between the HVRs as: (HC-FR1)-(HVR-H1)-(HC-FR2)-(HVR-H2)-(HC-FR3)-(HVR-H3)-(HC-FR4), and the light chain variable regions comprises one or more framework sequences juxtaposed between the HVRs as: (LC-FR1)-(HVR-L1)-(LC-FR2)-(HVR-L2)-(LC-FR3)-(HVR-L3)-(LC-FR4). In a still further aspect, the framework sequences are derived from human consensus framework sequences. In a still further aspect, the heavy chain framework sequences are derived from a Kabat subgroup I, II, or III sequence. In a still further aspect, the heavy chain framework sequence is a VH subgroup III consensus framework. In a still further aspect, one or more of the heavy chain framework sequences is the following:

| | | |
|---|---|---|
| HC-FR1 | EVQLVESGGGLVQPGGSLRLSCAASGFTFS | (SEQ ID NO:29) |
| HC-FR2 | WVRQAPGKGLEWVA | (SEQ ID NO:30) |
| HC-FR3 | RFTISADTSKNTAYLQMNSLRAEDTAVYYCAR | (SEQ ID NO:10) |
| HC-FR4 | WGQGTLVTVSS | (SEQ ID NO:27) |

In a still further aspect, the light chain framework sequences are derived from a Kabat kappa I, II, II or IV subgroup sequence. In a still further aspect, the light chain framework sequences are VL kappa I consensus framework. In a still further aspect, one or more of the light chain framework sequences is the following:

| | | |
|---|---|---|
| LC-FR1 | DIQMTQSPSSLSASVGDRVTITC | (SEQ ID NO:15) |
| LC-FR2 | WYQQKPGKAPKLLIY | (SEQ ID NO:16) |
| LC-FR3 | GVPSRFSGSGSGTDFTLTISSLQPEDFATYYC | (SEQ ID NO:17) |
| LC-FR4 | FGQGTKVEIK | (SEQ ID NO:28) |

In a still further specific aspect, the antibody further comprises a human or murine constant region. In a still further aspect, the human constant region is selected from the group consisting of IgG1, IgG2, IgG2, IgG3, IgG4. In a still further specific aspect, the human constant region is IgG1. In a still further aspect, the murine constant region is selected from the group consisting of IgG1, IgG2A, IgG2B, IgG3. In a still further aspect, the murine constant region if IgG2A. In a still further specific aspect, the antibody has reduced or minimal effector function. In a still further specific aspect the minimal effector function results from an "effector-less Fc mutation" or aglycosylation. In still a further embodiment, the effector-less Fc mutation is an N297A or D265A/N297A substitution in the constant region.

In yet another embodiment, provided is an anti-PD-Ll antibody comprising a heavy chain and a light chain variable region sequence, wherein:
(a) the heavy chain further comprises an HVR-H1, HVR-H2 and an HVR-H3 sequence having at least 85% sequence identity to GFTFSDSWIH (SEQ ID NO: 19), AWISPYGGSTYYADSVKG (SEQ ID NO:20) and RHWPGGFDY (SEQ ID NO:21), respectively, and/or
(b) the light chain further comprises an HVR-L1, HVR-L2 and an HVR-L3 sequence having at least 85% sequence identity to RASQDVSTAVA (SEQ ID NO:22), SASFLYS (SEQ ID NO:23) and QQYLYHPAT (SEQ ID NO:24), respectively.

In a specific aspect, the sequence identity is 86%, 87%, 88%, 89%, 90%, 91%, 92%, 93%, 94%, 95%, 96%, 97%, 98%, 99% or 100%.

In another aspect, the heavy chain variable region comprises one or more framework sequences juxtaposed between the HVRs as: (HC-FR1)-(HVR-H1)-(HC-FR2)-(HVR-H2)-(HC-FR3)-(HVR-H3)-(HC-FR4), and the light chain variable regions comprises one or more framework sequences juxtaposed between the HVRs as: (LC-FR1)-(HVR-L1)-(LC-FR2)-(HVR-L2)-(LC-FR3)-(HVR-L3)-(LC-FR4). In yet another aspect, the framework sequences are derived from human consensus framework sequences. In a still further aspect, the heavy chain framework sequences are derived from a Kabat subgroup I, II, or III sequence. In a still further aspect, the heavy chain framework sequence is a VH subgroup III consensus framework. In a still further aspect, one or more of the heavy chain framework sequences are set forth as SEQ ID NOs:8, 9, 10 and WGQGTLVTVSSASTK (SEQ ID NO:31).

In a still further aspect, the light chain framework sequences are derived from a Kabat kappa I, II, II or IV subgroup sequence. In a still further aspect, the light chain framework sequences are VL kappa I consensus framework. In a still further aspect, one or more of the light chain framework sequences are set forth as SEQ ID NOs: 15, 16, 17 and 18. In a still further specific aspect, the antibody further comprises a human or murine constant region. In a still further aspect, the human constant region is selected from the group consisting of IgG1, IgG2, IgG2, IgG3, IgG4. In a still further specific aspect, the human constant region is IgG1. In a still further aspect, the murine constant region is selected from the group consisting of IgG1, IgG2A, IgG2B, IgG3. In a still further aspect, the murine constant region if IgG2A. In a still further specific aspect, the antibody has reduced or minimal effector function. In a still further specific aspect the minimal effector function results from an "effector-less Fc mutation" or aglycosylation. In still a further embodiment, the effector-less Fc mutation is an N297A or D265A/N297A substitution in the constant region.

In a still further embodiment, provided is an isolated anti-PD-Ll antibody comprising a heavy chain and a light chain variable region sequence, wherein:
(a) the heavy chain sequence has at least 85% sequence identity to the heavy chain sequence: 
EVQLVESGGGLVQPGGSLRLSCAASGFTFSDSWIHWVRQAPGKGLEWVAWISPYGGSTYYA
DSVKGRFTISADTSKNTAYLQMNSLRAEDTAVYYCARRHWPGGFDYWGQGTLVTVSSASTK
(SEQ ID NO:26),
 or
(b) the light chain sequences has at least 85% sequence identity to the light chain sequence:

DIQMTQSPSSLSASVGDRVTITCRASQDVSTAVAWYQQKPGKAPKLLIYSASFLYSGVPSRFSG
SGSGTDFTLTISSLQPEDFATYYCQQYLYHPATFGQGTKVEIKR (SEQ ID NO:4).

In some embodiments, provided is an isolated anti-PD-Ll antibody comprising a heavy chain and a light chain variable region sequence, wherein the light chain variable region sequence has at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% sequence identity to the amino acid sequence of SEQ ID NO:4. In some embodiments, provided is an isolated anti-PD-Ll antibody comprising a heavy chain and a light chain variable region sequence, wherein the heavy chain variable region sequence has at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% sequence identity to the amino acid sequence of SEQ ID NO:26. In some embodiments, provided is an isolated anti-PD-Ll antibody comprising a heavy chain and a light chain variable region sequence, wherein the light chain variable region sequence has at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence identity to the amino acid sequence of SEQ ID NO:4 and the heavy chain variable region sequence has at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence identity to the amino acid sequence of SEQ ID NO:26. In some embodiments, one, two, three, four or five amino acid residues at the N-terminal of the heavy and/or light chain may be deleted, substituted or modified.

In a still further embodiment, provided is an isolated anti-PD-Ll antibody comprising a heavy chain and a light chain sequence, wherein:
(a) the heavy chain sequence has at least 85% sequence identity to the heavy chain sequence: 
EVQLVESGGGLVQPGGSLRLSCAASGFTFSDSWIHWVRQAPGKGLEWVAWISPYGGSTYYA
DSVKGRFTISADTSKNTAYLQMNSLRAEDTAVYYCARRHWPGGFDYWGQGTLVTVSSASTK
GPSVFPLAPSSKSTSGGTAALGCLVKDYFPEPVTVSWNSGALTSGVHTFPAVLQSSGLYSLSSV
VTVPSSSLGTQTYICNVNHKPSNTKVDKKVEPKSCDKTHTCPPCPAPELLGGPSVFLFPPKPKD
TLMISRTPEVTCVVVDVSHEDPEVKFNWYVDGVEVHNAKTKPREEQYASTYRVVSVLTVLH
QDWLNGKEYKCKVSNKALPAPIEKTISKAKGQPREPQVYTLPPSREEMTKNQVSLTCLVKGF
YPSDIAVEWESNGQPENNYKTTPPVLDSDGSFFLYSKLTVDKSRWQQGNVFSCSVMHEALHN
HYTQKSLSLSPG (SEQ ID NO:32),
 and/or
(b) the light chain sequences has at least 85% sequence identity to the light chain sequence:

DIQMTQSPSSLSASVGDRVTITCRASQDVSTAVAWYQQKPGKAPKLLIYSASFLYSGVPSRFSG
SGSGTDFTLTISSLQPEDFATYYCQQYLYHPATFGQGTKVEIKRTVAAPSVFIFPPSDEQLKSGT
ASVVCLLNNFYPREAKVQWKVDNALQSGNSQESVTEQDSKDSTYSLSSTLTLSKADYEKHK
VYACEVTHQGLSSPVTKSFNRGEC (SEQ ID NO:33).

In some embodiments, provided is an isolated anti-PD-Ll antibody comprising a heavy chain and a light chain sequence, wherein the light chain sequence has at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence of SEQ ID NO:33. In some embodiments, provided is an isolated anti-PD-Ll antibody comprising a heavy chain and a light chain sequence, wherein the heavy chain sequence has at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence of SEQ ID NO:32. In some embodiments, provided is an isolated anti-PD-Ll antibody comprising a heavy chain and a light chain sequence, wherein the light chain sequence has at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence of SEQ ID NO:33 and the heavy chain sequence has at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the amino acid sequence of SEQ ID NO:32.

In some embodiments, the isolated anti-PD-Ll antibody is aglycosylated. Glycosylation of antibodies is typically either N-linked or O-linked. N-linked refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-aceylgalactosamine, galactose, or xylose to a hydroxyamino acid, most commonly serine or threonine, although 5-hydroxyproline or 5-hydroxylysine may also be used. Removal of glycosylation sites form an antibody is conveniently accomplished by altering the amino acid sequence such that one of the above-described tripeptide sequences (for N-linked glycosylation sites) is removed. The alteration may be made by substitution of an asparagine, serine or threonine residue within the glycosylation site another amino acid residue (e.g., glycine, alanine or a conservative substitution).

In any of the embodiments herein, the isolated anti-PD-Ll antibody can bind to a human PD-L1, for example a human PD-L1 as shown in UniProtKB/Swiss-Prot Accession No.Q9NZQ7.1, or a variant thereof.

In a still further embodiment, provided is an isolated nucleic acid encoding any of the antibodies described herein. In some embodiments, the nucleic acid further comprises a vector suitable for expression of the nucleic acid encoding any of the previously described anti-PD-Ll antibodies. In a still further specific aspect, the vector is in a host cell suitable for expression of the nucleic acid. In a still further specific aspect, the host cell is a eukaryotic cell or a prokaryotic cell. In a still further specific aspect, the eukaryotic cell is a mammalian cell, such as Chinese hamster ovary (CHO) cell.

The antibody or antigen binding fragment thereof, may be made using methods known in the art, for example, by a process comprising culturing a host cell containing nucleic acid encoding any of the previously described anti-PD-Ll antibodies or antigen-binding fragment in a form suitable for expression, under conditions suitable to produce such antibody or fragment, and recovering the antibody or fragment.

### V. Pharmaceutical Formulations

Pharmaceutical formulations of an antagonist of an antagonist of B-RAF (e.g., vemurafenib), an antagonist of MEK *(e.g.,* cobimetinib) and/or a PD1 axis inhibitor *(e.g.,* atezolizumab) described herein may be prepared by formulating with one or more optional pharmaceutically acceptable carriers (Remington's Pharmaceutical Sciences 16th edition, Osol, A. Ed. (1980)), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as polysorbates (*e.g.,* TWEEN™), poloxamers (*e.g.,* PLURONICS™) or polyethylene glycol (PEG). The active pharmaceutical ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions.Exemplary pharmaceutically acceptable carriers herein further include insterstitial drug dispersion agents such as soluble neutral-active hyaluronidase glycoproteins (sHASEGP), for example, human soluble PH-20 hyaluronidase glycoproteins, such as rHuPH20 (HYLENEX®, Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases.

In particular, formulations to be used for *in vivo* administration must be sterile. Such sterilization is readily accomplished by filtration through sterile filtration membranes. The compounds ordinarily can be stored as a solid composition, a lyophilized formulation or as an aqueous solution.

The pharmaceutical compositions comprising an antagonist of B-RAF (e.g., vemurafenib), an antagonist of MEK *(e.g.,* cobimetinib) and/or a PD1 axis inhibitor *(e.g.,* atezolizumab) described herein can be formulated, dosed and administered in a fashion, *i.e*., amounts, concentrations, schedules, course, vehicles and route of administration, consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The "therapeutically effective amount" of the compound to be administered will be governed by such considerationsSuch techniques are disclosed in *Remington's Pharmaceutical Sciences.*

Sustained-release preparations comprising an antagonist of B-RAF (e.g., vemurafenib), an antagonist of MEK *(e.g.,* cobimetinib) and/or a PD1 axis inhibitor *(e.g.,* atezolizumab) described herein may be prepared. Suitable examples of sustained-release preparations include semipermeable matrices of solid hydrophobic polymers containing an antagonist of B-RAF (e.g., vemurafenib), an antagonist of MEK *(e.g.,* cobimetinib) and/or a PD1 axis inhibitor *(e.g.,* atezolizumab) described herein which matrices are in the form of shaped articles, e.g., films, or microcapsules. Examples of sustained-release matrices include polyesters, hydrogels (for example, poly(2-hydroxyethylmethacrylate), or poly(vinyl alcohol)), polylactides (US 3773919), copolymers of L-glutamic acid and gamma-ethyl-L-glutamate, non-degradable ethylene-vinyl acetate, degradable lactic acid-glycolic acid copolymers such as injectable microspheres composed of lactic acid-glycolic acid copolymer and leuprolide acetate (LUPRON DEPOT™) and poly-D-(-)-3-hydroxybutyric acid.

Formulations of an antagonist of B-RAF (*e.g.,* vemurafenib) or an antagonist of MEK (*e.g.,* cobimetinib) described herein may be suitable for oral administration, for example, may be prepared as discrete units such as pills, capsules, cachets or tablets each containing a predetermined amount of the antagonist of B-RAF *(e.g.,* vemurafenib) or the antagonist of MEK *(e.g.,* cobimetinib). For the B-RAF antagonist vemurafenib, the formulation may comprise a tablet core comprising one or more of hypromellose acetate succinate, croscarmellose sodium, colloidal silicon dioxide, magnesium stearate, and hydroxypropyl cellulose. In some embodiments, the tablet core may be coated with a coating comprising one or more of poly(vinyl alcohol), titanium dioxide, polyethylene glycol 3350, talc, and iron oxide red. In some embodiments, the B-RAF antagonist vemurafenib may be formulated in 240 mg tablets.. Vemurafenib has the chemical name propane-1-sulfonic acid {3-[5-(4-chlorophenyl)-1H-pyrrolo[2,3-b]pyridine-3-carbonyl]-2,4-difluoro-phenyl}-amide. For the antagonist of MEK (e.g., cobimetinib), the formulation may comprise tablet core comprising one or more of microcrystalline cellulose, lactose monohydrate, croscarmellose sodium, magnesium stearate. In some embodiments, the table core may be coated with a coating comprising one or more of comprising polyvinyl alcohol, titanium dioxide, polyethylene. In some embodiments, the MEK antagonist cobimetinib may be formulated in 20 mg tablets. In some embodiments, the 20 mg tablets contain 22 mg of cobimetinib fumarate, which corresponds to 20 mg of the cobimetinib free base. Formulations of a PD1 axis inhibitor (e.g., atezolizumab) described herein may be suitable for IV administration.

The formulations may be packaged in unit-dose or multi-dose containers, for example sealed ampoules and vials, and may be stored in a freeze-dried (lyophilized) condition requiring only the addition of the sterile liquid carrier, for example water, for injection immediately prior to use. Extemporaneous injection solutions and suspensions are prepared from sterile powders, granules and tablets of the kind previously described. Preferred unit dosage formulations are those containing a daily dose or unit daily sub-dose, as herein above recited, or an appropriate fraction thereof, of the active ingredient.

### VI. Articles of Manufacture

In another aspect of the invention, an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, IV solution bags, blister pack, etc. The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper pierceable by a hypodermic injection needle). At least one active agent in the composition is an antagonist of B-RAF (e.g., vemurafenib), an antagonist of MEK (*e.g.,* cobimetinib) and/or a PD1 axis inhibitor (*e.g.,* atezolizumab) described herein. The label or package insert indicates that the composition is used for treating the condition of choice. Moreover, the article of manufacture may comprise (a) a first container with a composition contained therein, wherein the composition comprises an antagonist of B-RAF (e.g., vemurafenib); and (b) a second container with a composition contained therein, wherein the composition comprises a PD1 axis inhibitor (e.g., atezolizumab). Moreover, the article of manufacture may comprise (a) a first container with a composition contained therein, wherein the composition comprises an antagonist of B-RAF (e.g., vemurafenib); (b) a second container with a composition contained therein, wherein the composition comprises an antagonist of MEK *(e.g.,* cobimetinib); and (c) a third container with a composition contained therein, wherein the composition comprises a PD1 axis inhibitor (e.g., atezolizumab).

The article of manufacture in this embodiment of the invention may further comprise a package insert indicating that the compositions can be used to treat a particular condition such as cancer or melanoma (e.g., V600 mutant unresectable or metastatic melanoma). The package insert may refer to instructions customarily included in commercial packages of therapeutic products that contain information about the indications, usage, dosage, administration, contraindications and/or warnings concerning the use of such therapeutic products. Alternatively, or additionally, the article of manufacture may further comprise a second (or third or fourth) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

Although the foregoing invention has been described in some detail by way of illustration and example for purposes of clarity of understanding, the descriptions and examples should not be construed as limiting the scope of the invention. The disclosures of all patent and scientific literature cited herein are expressly incorporated in their entirety by reference.

### EXAMPLES

The following are examples of methods and compositions of the invention. It is understood that various other embodiments may be practiced, given the general description provided above.

### Example 1

### Materials and Methods

*Patient Inclusion Criteria:* Patients must meet the following criteria to be eligible for study entry: signed Informed Consent Form, age ≥ 18 years, and histologic or cytologic documentation of metastatic or Stage IIIc unresectable melanoma, with a BRAFV600 mutation (any missense type) as assessed by a BRAF V600 mutation test performed in a Clinical Laboratory Improvement Act (CLIA)-certified laboratory or equivalent. Origin of the primary tumor may be of skin, mucosal, or acral locations but not of uveal origin. Patients having an unknown primary tumor may be eligible if uveal melanoma can be ruled out and pending discussion with the Medical Monitor. ECOG performance status of 0 or 1, adequate hematologic and end-organ function, defined by the following laboratory results obtained within 14 days prior to Day 1: Neutrophils (ANC ≥ 1500 cells/µL), WBC counts >2500 cells/µL and < 15,000 cells/µL, Lymphocyte count ≥ 500 cells/µL (or within local laboratory normal limits), Platelet count ≥ 100,000 cells/µL, Hemoglobin ≥ 9.0 g/dL, total bilirubin ≤ 1.5 ×ULN with the following exception: Patients with known Gilbert disease who have serum bilirubin level ≤ 3 × ULN may be enrolled, AST and ALT ≤ 2.0 ×ULN, ALP ≤ 2.5 × ULN with the following exception: Patients with documented liver or bone metastases: ALP ≤ 5×ULN, Creatinine clearance ≥ 30 mL/min, INR and aPTT ≤ 1.5 × ULN within 14 days prior to Day 1, *and* measurable disease per RECIST v1.1.

Patient Exclusion Criteria: Patients who meet any of the following criteria will be excluded from study entry: receipt of prior systemic anti-cancer therapy (e.g., biologic or other targeted therapy, chemotherapy, investigational anti-cancer agents, or hormonal therapy) for unresectable, locally advanced, or metastatic melanoma, except for: IFN therapy in the adjuvant setting, discontinued at least 28 days prior to Day 1, IL-2 therapy, discontinued at least 28 days prior to Day 1, vaccine therapies, if discontinued at least 28 days prior to Day 1, herbal therapy intended as anti-cancer therapy must be discontinued ≥ 7 days prior to Day 1, receipt of prior immunomodulatory agents, including PD-1 or PD-L1 targeted therapy or CTLA-4 targeted therapy, including ipilimumab, with the above exceptions, as indicated in the previous exclusion criterion above, receipt of prior MAPK inhibitor pathway agents, including MEK kinase inhibitor and BRAF kinase inhibitor, major surgical procedure within 28 days prior to Day 1 or anticipation of need for a major surgical procedure during the course of the study, radiotherapy ≤ 7 days prior to Day 1, adverse events from prior anti-cancer therapy that have not resolved to Grade ≤1 except for alopecia, current severe, uncontrolled systemic disease (including but not limited to clinically significant cardiovascular, pulmonary, or renal disease) excluding cancer, known clinically significant liver disease, including active viral, alcoholic, or other hepatitis, cirrhosis, fatty liver, and inherited liver disease, active or untreated CNS metastases as determined by computed tomography (CT) or magnetic resonance imaging (MRI) evaluation during screening and prior assessments for CNS metastases. Note: Patients with a history of treated asymptomatic CNS metastases are eligible, provided they meet all of the following criteria: No metastases to brain stem, midbrain, pons, medulla, or within 10 mm of the optic apparatus (optic nerves and chiasm), leptomeningeal disease is also excluded, radiographic demonstration of improvement upon the completion of CNS-directed therapy and no evidence of interim progression between the completion of CNS-directed therapy and the screening radiographic study, no history of intracranial hemorrhage, no ongoing requirement for dexamethasone as therapy for CNS disease; anticonvulsants at a stable dose allowed, no stereotactic radiation or whole-brain radiation within 28 days prior to Day 1, screening CNS radiographic study ≥ 4 weeks since completion of radiotherapy and ≥ 2 weeks since discontinuation of corticosteroids, patients with active malignancy (other than BRAF-mutated melanoma) or a previous malignancy within the past 3 years are excluded except for patients with resected melanoma, resected BCC, resected cuSCC, resected melanoma in situ, resected carcinoma in situ of the cervix, resected carcinoma in situ of the breast, or other malignancies with similar outcome (depending on discussion with the medical monitor), prior allogeneic bone marrow transplantation or prior solid organ transplantation, history of autoimmune disease, including but not limited to myasthenia gravis, myositis, autoimmune hepatitis, systemic lupus erythematosus, rheumatoid arthritis, inflammatory bowel disease, vascular thrombosis associated with antiphospholipid syndrome, Wegener's granulomatosis, Sjögren's syndrome, Guillain-Barre syndrome, multiple sclerosis, vasculitis, or glomerulonephritis (patients with a history of autoimmune-related hypothyroidism on a stable dose of thyroid-replacement hormone may be eligible for this study and patients with controlled Type 1 diabetes mellitus on a stable insulin regimen may be eligible for this study.), history of idiopathic pulmonary fibrosis (including pneumonitis), risk of pulmonary toxicity, or evidence of active pneumonitis on screening chest CT scan, history of HTV infection, patients with active hepatitis B (defined as having a positive hepatitis B surface antigen [HBsAg] test at screening), *active hepatitis C, tuberculosis, etc.*

Study Design: The open-label, multicenter, Phase Ib, study of atezolizumab in combination with two targeted treatments (vemurafenib monotherapy and vemurafenib + cobimetinib combined therapy) in previously untreated patients with BRAFV600 mutation-positive metastatic melanoma. Dose levels of atezolizumab administered intravenously were fixed at either 15 or 20 mg/kg q3w, or at a fixed dose of either 1200 mg q3w or 800 mg every 2 weeks (q2w), depending on the arm/cohort. For targeted treatments, all vemurafenib dosing will be PO BID, and all cobimetinib dosing will be PO QD on a 21 days on/7 days off schedule. Schedules may contain a run-in period consisting of targeted treatment at starting doses of 960 mg vemurafenib with or without 60 mg cobimetinib prior to initiation of combination treatment with atezolizumab. Initial cohort starting dose levels of targeted treatments during the combination treatment period with targeted agents will be at 720 mg for vemurafenib and 60 mg for cobimetinib (based on results from the ongoing GP28363 study of cobimetinib + atezolizumab).

Abbreviations: A= atezolizumab; BID= twice daily; C = cobimetinib; MTD = maximum tolerated dose; q2w= every 2 weeks; q3w= every 3 weeks; QD = once daily; V = vemurafenib. Note: All V dosing is BID; all C dosing is QD on a 21 days on/7 days off schedule; A dosing is 15 mg/kg q3w or 1200 mg q3w with V only and 800 mg q2w with V +C. Note that C and V are given via oral dosing while A is administered by IV.
Cohort 1: V at 720 mg BID + A at 20 mg/kg q3w concurrent start. Initially proposed no run-in period; however, a run-in period was introduced following toxicity observed in concurrent start of V +A in Cohort 1 (although no DLT's were observed).
Cohort 2: 56-day run in with V at 960 mg BID for 49 days then V at 720 mg BID for 7 days followed by: V at 720 mg BID +A at 15 mg/kg q3w. The length of the run-in period in Cohort 4 (28 days) was selected on the basis of the tolerability of regimens tested in Cohorts 2 (56 day run-in) and Cohort 3 (28 day run-in).
Cohort 3: 28 day run in with V at 960 mg BID for 21 days then V at 720 mg BID for 7 days followed by: V at 720 mg BID + A at 1200 mg q3w.
Cohort 4: 28-day run-in V + C (V 21 days at 960 mg BID and 7 days V at 720 mg BID + C 60 mg QD 21/7) followed by: V at 720 mg BID + C at 60 mg QD 21/7 + A at 800 mg q2w on 28 day cycle. The length of the run-in period in Cohort 4 (28 days) was selected on the basis of the tolerability of regimens tested in Cohorts 2 (56 day run-in) and Cohort 3 (28 day run-in).

Expansion Cohort A: V + A. Patients are enrolling into the expansion of the Cohort 3 regimen: 28-day vemurafenib-only run-in (21 days at 960 mg BID and 7 days at 720 mg BID), followed by 21 day cycles including treatment with V at 720 mg BID and A dosed at 1200 mg q3w.
Expansion Cohort B: V+C+A. Patients are enrolling in the expansion of the Cohort 4 regimen: 28-day run-in V + C (V 21 days at 960 mg BID and 7 days V at 720 mg BID + C at 60 mg QD 21/7), followed by: V at 720 mg BID + C at 60 mg QD 21/7 and A at 800 mg q2w in 28-day cycles.
Patients in any of the above cohorts may have dose modification based on protocol requirements for adverse events for vemurafenib dose reduction to 480 mg BID and/or cobimetinib reduction to 40 mg QD 21/7.

### Results

Table 1 below provides the baseline characteristics of patients. This Phase Ib dose-escalation study demonstrates promising anti-tumor activity and tolerability of vermurafenib + atezolizumab combination therapy in treatment of BRAFV600 metastatic melanoma. The treatment resulted in 16/16 (100%) patients evaluable for tumor response had SLD reduction in target lesions. The median duration of response was 20.9 mo and median progression-free survival was 10.9 mo for all patients.

Greater tolerability was observed with a staggered vermurafenib + atezolizumab start vs concurrent start. The objective response rate of concurrent administration of vemurafenib and atezolizumab in Cohort 1 resulted in 1/3 complete responses and 0/3 partial responses for an overall response rate of 33%. The objective response rate of the 56 day run in of vemurafenib prior to administration of vemurafenib and atezolizumab in Cohort 2 resulted in 1/8 complete responses and 5/8 partial responses for an overall response rate of 75%. The objective response rate of the 28 day run in of vemurafenib prior to administration of vemurafenib and atezolizumab in Cohort 3 resulted in 1/6 complete responses and 5/6 partial responses for an overall response rate of 100%. A higher ORR in Cohort 3 may indicate that the ideal schedule for run-in would 28 days (which is included in both expansion cohorts). As shown in Table 2 below, overall a staggered dosing was better tolerated compared concurrent dosing.

Tissue samples were stained for CD8+ T-cells as well as PD-L1 expression. Treatment with vemurafenib alone increased immune tumor CD8+ T-cell infiltration (data not shown). Treatment with either vemurafenib alone or vemurafenib in combination with atezolizumab increased expression of PD-L1 on tumor cells as well as tumor-infiltrating immune cells (data not shown).

| | **All (N = 17)** | **Cohort 1 (n = 3)** | **Cohort 2 (n = 8)** | **Cohort 3 (n = 6)** |
|---|---|---|---|---|
| Median age, y | 53 | 48 | 59 | 52 |
| 18-65 y | 82% | 100% | 75% | 83% |
| Male | 65% | 100% | 50% | 67% |
| ECOG score, 0/1 | 82%/18% | 100%/0% | 75%/25% | 83%/17% |

| Histology subtypes, % | | | | |
|---|---|---|---|---|
| Nodular | 59% | 67% | 63% | 50% |
| Superficial spreading | 23% | 0% | 25% | 33% |
| Other / missing | 18% | 33% | 12% | 17% |

| Stage at screening, n (%) | | | | |
|---|---|---|---|---|
| IIIc | 1 (5.9%) | 0 (0%) | 1 (12.5%) | 0 (0%) |
| MIA | 3 (17.6%) | 0 (0%) | 1 (12.5%) | 2 (33.3%) |
| M1B | 3 (17.6%) | 0 (0%) | 2 (25.0%) | 1 (16.7%) |
| M1C | 10 (58.8%) | 3 (100%) | 4 (50.0%) | 3 (50.0%) |
| Median time from first diagnosis to first treatment, mo (range) | 22.5 (0.2-415.7) | 4.2 (2.4-7.8) | 46.3 (0.2-415.7) | 24.3 (2.4-176.5) |
| | | | | |

| | **All (N = 17)** | Concurrent **A +V** | **Staggered A+V** | |
|---|---|---|---|---|
| | | **C1** (n = 3) | **C2** (n = 8) | C3 (n = 6) |
| Median Safety Follow-up, mo | 12.3 | 6.5 | 10.6 | 14.2 |
| All Treatment-Emergent AEs | 100% | 100% | 100% | 100% |
| Grade 3 A-related AEs | 41% | 67% | 38% | 33% |
| Grade 3 V-related AEs | 59% | 100% | 50% | 50% |

Initial early findings in Cohort 4 with a run-in of vemurafenib and cobimetinib followed by administration of vemurafenib, cobimetinib and atezolizumab in ten patients(as of September 2015), showed that eight patients had a total of twelve G3 related AE's and one G4 related AE which were manageable and generally reversible. Six of the twelve Grade 3 related events were in the run-in period (prior to the addition of atezolizumab). The events included rash, photosensitivity, diarrhea, anemia, cellulitis and LFT elevations. Specifically, three patients had Grade 3 ALT or AST Elevations: 2 patients during the run in period (which resolved with dose modification), and one patient during cycle 2 which resolved with dose modification including vemurafenib dose reduction to 480 mg BID and/or cobimetinib reduction to 40 mg QD 21/7. There was one patient who discontinued study drug treatment due to AE's of G3 AST/ALT/Bilirubin and G4 ALT, which were attributed to atezolizumab and vemurafenib. Of the nine evaluable patients treated with vemurafenib and cobimetinib prior to administration of vemurafenib, cobimetinib and atezolizumab in Cohort 4 as of September 2015, 7/9 patients best response was a partial response, 1/9 best response was stable disease, and 1/9 best response was progressive disease. A ninety percent disease control rate was seen with vemurafenib, cobimetinib and atezolizumab in early data. This study is ongoing and will enroll 20 total patients to be treated with the triple combination of vemurafenib, cobimetinib and atezolizumab and 10 additional patients to be treated with vemurafenib and atezolizumab in the expansion cohorts.

## Claims

1. The triple combination of active ingredients with the INN's vemurafenib, cobimetinib and atezolizumab for use in treating cancer in an individual, **characterized by** first administering to the individual an effective amount of vemurafenib and cobimetinib and second administering to the individual an effective amount of vemurafenib, cobimetinib and atezolizumab.

2. The triple combination of active ingredients with the INN's vemurafenib, cobimetinib and atezolizumab for use in a method of increasing efficacy of a cancer treatment, the method comprising first administering to the individual an effective amount of vemurafenib and cobimetinib and second administering to the individual an effective amount of vemurafenib, cobimetinib and atezolizumab.

3. The triple combination for use according to claims 1 or 2, wherein the first administration and second administration of vemurafenib is at a dosage of about 960 mg twice daily, about 720 mg twice daily, and/or about 480 mg twice daily.

4. The triple combination for use according to claim 3, wherein the first administration of vemurafenib is at a greater dosage then the second administration of vemurafenib.

5. The triple combination for use according to claim 3, wherein the first administration of vemurafenib comprises a first dosage and a second dosage of vemurafenib and the first dosage is greater than the second dosage.

6. The triple combination for use according to any one of claims 1-5, wherein the first administration of vemurafenib is about 28 days or about 56 days.

7. The triple combination for use according to claim 6, wherein the first administration of vemurafenib comprises a first dosage and a second dosage of vemurafenib, the first dosage is greater than the second dosage, and the first dosage is administered for 21 days and the second dosage is administered for 7 days.

8. The triple combination for use according to any one of claims 1-7, wherein vemurafenib is administered orally.

9. The triple combination for use according to any one of claims 1 to 8, wherein atezolizumab is administered at a dosage of about 15 mg/kg q3w, about 20 mg/kg q3w, about 800 mg q2w, or about 1200 mg q3w.

10. The triple combination for use according to any one of claims 1-9, wherein atezolizumab is administered intravenously.

11. The triple combination for use according to any one of claims 1-10, wherein the first administration and second administration of cobimetinib is at a dosage of about 60 mg daily on a 21 days on/7 days off schedule or about 40 mg daily on a 21 days on/7 days off schedule.

12. The triple combination for use according to any one of claims 1-10, wherein the first administration of cobimetinib is about 28 day schedule.

13. The triple combination for use according to any one of claims 1-11, wherein cobimetinib is administered orally.

14. The triple combination for use according to any one of claims 1-13, wherein the cancer is melanoma.

15. The triple combination for use according to claim 14, wherein the melanoma is unresectable or metastatic melanoma.

16. The triple combination for use according to any one of claims 14-15, wherein the melanoma is B-RAF V600 mutant melanoma.

17. The triple combination for use according to claim 16, wherein the melanoma is B-RAF V600E mutant melanoma or B-RAF V600K mutant melanoma.

18. The triple combination of active ingredients with the INN's vemurafenib, cobimetinib and atezolizumab for use in treating melanoma in an individual, the use comprising first administering to the individual vemurafenib and cobimetinib on a 28 day schedule, wherein vemurafenib is administered at a dosage of 960 mg twice a day for 21 days and then 720 mg twice a day for 7 days of the 28 day schedule and cobimetinib is administered at a dosage of 60 mg daily for 21 days and 7 days off of the 28 day schedule and second administering to the individual vemurafenib, cobimetinib, and atezolizumab, wherein vemurafenib is administered at a dosage of 720 mg twice a day, cobimetinib is administered at a dosage of 60 mg daily for 21 days on and 7 days off, and atezolizumab is administered at a dosage of 800 mg q2w.

19. The triple combination for use according to claim 18, wherein the vemurafenib is administered orally as a tablet.

20. The triple combination for use according to any one of claims 18-19, wherein the cobimetinib is administered orally as a tablet.

21. The triple combination for use according to any one of claims 18-20, wherein the atezolizumab is administered intravenously.

22. The triple combination for use according to any one of claims 18-21, wherein the cancer is melanoma.

23. The triple combination for use according to claim 22, wherein the melanoma is unresectable or metastatic melanoma.

24. The triple combination for use according to any one of claims 22-23, wherein the melanoma is B-RAF V600 mutant melanoma.

25. The triple combination for use according to claim 24, wherein the B-RAF V600 mutant melanoma is B-RAF V600E mutant melanoma or B-RAF V600K mutant melanoma.

## Patentansprüche

1. Dreifachkombination von Wirkstoffen mit den INNs Vemurafenib, Cobimetinib und Atezolizumab zur Verwendung bei der Behandlung von Krebs bei einem Individuum, **gekennzeichnet durch** ein erstes Verabreichen einer wirksamen Menge von Vemurafenib und Cobimetinib an das Individuum und ein zweites Verabreichen einer wirksamen Menge von Vemurafenib, Cobimetinib und Atezolizumab an das Individuum.

2. Dreifachkombination von Wirkstoffen mit den INNs Vemurafenib, Cobimetinib und Atezolizumab zur Verwendung in einem Verfahren zum Steigern der Wirksamkeit einer Krebsbehandlung, wobei das Verfahren ein erstes Verabreichen einer wirksamen Menge von Vemurafenib und Cobimetinib an das Individuum und ein zweites Verabreichen einer wirksamen Menge von Vemurafenib, Cobimetinib und Atezolizumab an das Individuum umfasst.

3. Dreifachkombination zur Verwendung nach den Ansprüchen 1 oder 2, wobei die erste Verabreichung und die zweite Verabreichung von Vemurafenib in einer Dosierung von etwa 960 mg zweimal täglich, etwa 720 mg zweimal täglich und/oder etwa 480 mg zweimal täglich erfolgen.

4. Dreifachkombination zur Verwendung nach Anspruch 3, wobei die erste Verabreichung von Vemurafenib in einer höheren Dosierung als die zweite Verabreichung von Vemurafenib erfolgt.

5. Dreifachkombination zur Verwendung nach Anspruch 3, wobei die erste Verabreichung von Vemurafenib eine erste Dosierung und eine zweite Dosierung von Vemurafenib umfasst und die erste Dosierung höher ist als die zweite Dosierung.

6. Dreifachkombination zur Verwendung nach einem der Ansprüche 1-5, wobei die erste Verabreichung von Vemurafenib etwa 28 Tage oder etwa 56 Tage beträgt.

7. Dreifachkombination zur Verwendung nach Anspruch 6, wobei die erste Verabreichung von Vemurafenib eine erste Dosierung und eine zweite Dosierung von Vemurafenib umfasst, wobei die erste Dosierung höher ist als die zweite Dosierung, und die erste Dosierung über 21 Tage verabreicht wird und die zweite Dosierung über 7 Tage verabreicht wird.

8. Dreifachkombination zur Verwendung nach einem der Ansprüche 1-7, wobei Vemurafenib oral verabreicht wird.

9. Dreifachkombination zur Verwendung nach einem der Ansprüche 1 bis 8, wobei Atezolizumab in einer Dosierung von etwa 15 mg/kg alle drei Wochen (q3w), etwa 20 mg/kg q3w, etwa 800 mg alle zwei Wochen (q2w) oder etwa 1.200 mg q3w verabreicht wird.

10. Dreifachkombination zur Verwendung nach einem der Ansprüche 1-9, wobei Atezolizumab intravenös verabreicht wird.

11. Dreifachkombination zur Verwendung nach einem der Ansprüche 1-10, wobei die erste Verabreichung und die zweite Verabreichung von Cobimetinib in einer Dosierung von etwa 60 mg täglich nach einem Zeitplan von 21 Tagen Einnahme/7 Tagen Nichteinnahme oder etwa 40 mg täglich nach einem Zeitplan von 21 Tagen Einnahme/7 Tagen Nichteinnahme erfolgen.

12. Dreifachkombination zur Verwendung nach einem der Ansprüche 1-10, wobei die erste Verabreichung von Cobimetinib nach einem Zeitplan über etwa 28 Tage erfolgt.

13. Dreifachkombination zur Verwendung nach einem der Ansprüche 1-11, wobei Cobimetinib oral verabreicht wird.

14. Dreifachkombination zur Verwendung nach einem der Ansprüche 1-13, wobei der Krebs Melanom ist.

15. Dreifachkombination zur Verwendung nach Anspruch 14, wobei das Melanom nicht resezierbares oder metastatisches Melanom ist.

16. Dreifachkombination zur Verwendung nach einem der Ansprüche 14-15, wobei das Melanom ein Melanom mit B-RAF V600-Mutation ist.

17. Dreifachkombination zur Verwendung nach Anspruch 16, wobei das Melanom ein Melanom mit B-RAF V600E-Mutation oder ein Melanom mit B-RAF V600K-Mutation ist.

18. Dreifachkombination von Wirkstoffen mit den INNs Vemurafenib, Cobimetinib und Atezolizumab zur Verwendung bei der Behandlung von Melanom bei einem Individuum, wobei die Verwendung ein erstes Verabreichen von Vemurafenib und Cobimetinib nach einem 28-Tage-Zeitplan an das Individuum, wobei Vemurafenib in einer Dosierung von 960 mg zweimal am Tag über 21 Tage und dann 720 mg zweimal am Tag über 7 Tage des 28-Tage-Zeitplanes verabreicht wird und Cobimetinib in einer Dosierung von 60 mg täglich über 21 Tage und 7 Tage Nichteinnahme des 28-Tage-Zeitplanes verabreicht wird, und ein zweites Verabreichen von Vemurafenib, Cobimetinib und Atezolizumab an das Individuum, wobei Vemurafenib in einer Dosierung von 720 mg zweimal am Tag verabreicht wird, Cobimetinib in einer Dosierung von 60 mg täglich über 21 Tage Einnahme und 7 Tage Nichteinnahme verabreicht wird und Atezolizumab in einer Dosierung von 800 mg q2w verabreicht wird, umfasst.

19. Dreifachkombination zur Verwendung nach Anspruch 18, wobei das Vemurafenib oral als eine Tablette verabreicht wird.

20. Dreifachkombination zur Verwendung nach einem der Ansprüche 18-19, wobei das Cobimetinib oral als eine Tablette verabreicht wird.

21. Dreifachkombination zur Verwendung nach einem der Ansprüche 18-20, wobei das Atezolizumab intravenös verabreicht wird.

22. Dreifachkombination zur Verwendung nach einem der Ansprüche 18-21, wobei der Krebs Melanom ist.

23. Dreifachkombination zur Verwendung nach Anspruch 22, wobei das Melanom nicht resezierbares oder metastatisches Melanom ist.

24. Dreifachkombination zur Verwendung nach einem der Ansprüche 22-23, wobei das Melanom ein Melanom mit B-RAF V600-Mutation ist.

25. Dreifachkombination zur Verwendung nach Anspruch 24, wobei das Melanom mit B-RAF V600-Mutation ein Melanom mit B-RAF V600E-Mutation oder ein Melanom mit B-RAF V600K-Mutation ist.

## Revendications

1. Trithérapie de principes actifs ayant les DCI vémurafénib, cobimétinib et atézolizumab pour une utilisation dans le traitement du cancer chez un individu, **caractérisée par** une première administration à l'individu d'une quantité efficace de vémurafénib et de cobimétinib et une seconde administration à l'individu d'une quantité efficace de vémurafénib, de cobimétinib et d'atézolizumab.

2. Trithérapie de principes actifs ayant les DCI vémurafénib, cobimétinib et atézolizumab pour une utilisation dans une méthode d'augmentation de l'efficacité d'un traitement du cancer, la méthode comprenant une première administration à l'individu d'une quantité efficace de vémurafénib et de cobimétinib et une seconde administration à l'individu d'une quantité efficace de vémurafénib, de cobimétinib et d'atézolizumab.

3. Trithérapie pour une utilisation selon les revendications 1 ou 2, dans laquelle la première administration et la seconde administration de vémurafénib sont à une posologie d'environ 960 mg deux fois par jour, d'environ 720 mg deux fois par jour et/ou d'environ 480 mg deux fois par jour.

4. Trithérapie pour une utilisation selon la revendication 3, dans laquelle la première administration de vémurafénib est à une posologie supérieure à la seconde administration de vémurafénib.

5. Trithérapie pour une utilisation selon la revendication 3, dans laquelle la première administration de vémurafénib comprend une première posologie et une seconde posologie de vémurafénib et la première posologie est supérieure à la seconde posologie.

6. Trithérapie pour une utilisation selon l'une quelconque des revendications 1 à 5, dans laquelle la première administration de vémurafénib est d'environ 28 jours ou d'environ 56 jours.

7. Trithérapie pour une utilisation selon la revendication 6, dans laquelle la première administration de vémurafénib comprend une première posologie et une seconde posologie de vémurafénib, la première posologie est supérieure à la seconde posologie et la première posologie est administrée pendant 21 jours et la seconde posologie est administrée pendant 7 jours.

8. Trithérapie pour une utilisation selon l'une quelconque des revendications 1 à 7, dans laquelle le vémurafénib est administré par voie orale.

9. Trithérapie pour une utilisation selon l'une quelconque des revendications 1 à 8, dans laquelle l'atézolizumab est administré à une posologie d'environ 15 mg/kg toutes les 3 semaines, d'environ 20 mg/kg toutes les 3 semaines, d'environ 800 mg toutes les 2 semaines ou d'environ 1 200 mg toutes les 3 semaines.

10. Trithérapie pour une utilisation selon l'une quelconque des revendications 1 à 9, dans laquelle l'atézolizumab est administré par voie intraveineuse.

11. Trithérapie pour une utilisation selon l'une quelconque des revendications 1 à 10, dans laquelle la première administration et la seconde administration de cobimétinib sont à une posologie d'environ 60 mg par jour sur un programme de 21 jours de traitement/7 jours d'arrêt ou d'environ 40 mg par jour sur un programme de 21 jours de traitement/7 jours d'arrêt.

12. Trithérapie pour une utilisation selon l'une quelconque des revendications 1 à 10, dans laquelle la première administration de cobimétinib est un programme d'environ 28 jours.

13. Trithérapie pour une utilisation selon l'une quelconque des revendications 1 à 11, dans laquelle le cobimétinib est administré par voie orale.

14. Trithérapie pour une utilisation selon l'une quelconque des revendications 1 à 13, dans laquelle le cancer est un mélanome.

15. Trithérapie pour une utilisation selon la revendication 14, dans laquelle le mélanome est un mélanome non résécable ou métastatique.

16. Trithérapie pour une utilisation selon l'une quelconque des revendications 14 à 15, dans laquelle le mélanome est un mélanome à mutation B-RAF V600.

17. Trithérapie pour une utilisation selon la revendication 16, dans laquelle le mélanome est un mélanome à mutation B-RAF V600E ou un mélanome à mutation B-RAF V600K.

18. Trithérapie de principes actifs ayant les DCI vémurafénib, cobimétinib et atézolizumab pour une utilisation dans le traitement d'un mélanome chez un individu, l'utilisation comprenant une première administration à l'individu de vémurafénib et de cobimétinib sur un programme de 28 jours, dans laquelle le vémurafénib est administré à une posologie de 960 mg deux fois par jour pendant 21 jours et ensuite 720 mg deux fois par jour pendant 7 jours du programme de 28 jours et le cobimétinib est administré à une posologie de 60 mg une fois par jour pendant 21 jours et 7 jours d'arrêt du programme de 28 jours et une seconde administration à l'individu de vémurafénib, de cobimétinib et d'atézolizumab, dans laquelle le vémurafénib est administré à une posologie de 720 mg deux fois par jour, le cobimétinib est administré à une posologie de 60 mg une fois par jour pendant 21 jours et 7 jours d'arrêt et l'atézolizumab est administré à une posologie de 800 mg toutes les 2 semaines.

19. Trithérapie pour une utilisation selon la revendication 18, dans laquelle le vémurafénib est administré par voie orale sous la forme d'un comprimé.

20. Trithérapie pour une utilisation selon l'une quelconque des revendications 18 à 19, dans laquelle le cobimétinib est administré par voie orale sous la forme d'un comprimé.

21. Trithérapie pour une utilisation selon l'une quelconque des revendications 18 à 20, dans laquelle l'atézolizumab est administré par voie intraveineuse.

22. Trithérapie pour une utilisation selon l'une quelconque des revendications 18 à 21, dans laquelle le cancer est un mélanome.

23. Trithérapie pour une utilisation selon la revendication 22, dans laquelle le mélanome est un mélanome non résécable ou métastatique.

24. Trithérapie pour une utilisation selon l'une quelconque des revendications 22 à 23, dans laquelle le mélanome est un mélanome à mutation B-RAF V600.

25. Trithérapie pour une utilisation selon la revendication 24, dans laquelle le mélanome à mutation B-RAF V600 est un mélanome à mutation B-RAF V600E ou un mélanome à mutation B-RAF V600K.
